# EUROPEAN PATENT APPLICATION

(11) **EP 4 650 006 A2**
(43) Date of publication of application: **19.11.2025**
(21) Application number: 25206294.8
(22) Date of filing: 07.01.2022
(51) Int. Cl.: A61P 35/00

(54) **USE OF A KRAS G12C INHIBITOR IN TREATING CANCERS**

(30) Priority: 08.01.2021 US 202163135449 P; 17.05.2021 US 202163189625 P
(62) Divisional of application: 22703115.0
(71) Applicant: Amgen Inc., Thousand Oaks, CA 91320-1799 (US)
(72) Inventor: ANG, Agnes L., Thousand Oaks, California (US); HENARY, Haby, Thousand Oaks, California (US); NGARMCHAMNANRITH, Gataree, Thousand Oaks, California (US)
(74) Representative: Murgitroyd & Company

(57) **Abstract**

The present disclosure provides uses for a KRAS^{G12C} inhibitor, such as the compound of Formula I (AMG 510, sotorasib) in treating cancers, such as non-small cell lung cancer, in subjects with certain characteristics.

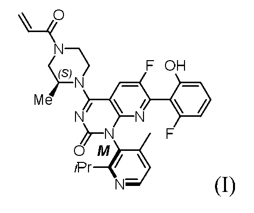

## Description

### CROSS-REFERENCE TO RELATED APPLICATIONS

This application claims the benefit of U.S. Provisional Patent Application No. 63/135,449, filed January 8, 2021, and U.S. Provisional Patent Application No. 63/189,625, filed May 17, 2021, each of which is incorporated herein by reference in its entirety.

### FIELD

The present disclosure provides uses for a KRAS^{G12C} inhibitor, such as the compound of Formula I (AMG 510, sotorasib) in treating cancers, such as non-small cell lung cancer, in subjects with certain characteristics.

### BACKGROUND

Lung cancer is the leading cause of cancer death, with more than 80% of all lung cancer cases classified as non-small cell lung cancer (NSCLC). Worldwide, lung cancer (small cell and non-small cell) is the most common cancer occurring in both men and women, with an estimated 2.09 million cases in 2018 (World Health Organization Statistics, 2018). In 2018, more than 250000, 470039, and 1225000 new cases of lung cancer were reported in North America, Europe, and Asia, respectively. The estimated number of deaths from lung cancer in 2018 was 173278 in North America, 387913 in Europe, and 1068862 in Asia (Globocan - Lung Cancer, 2018). Advanced NSCLC (stage IIIB and IV) is a serious and life-threatening disease, with a 5-year survival rate of 5.2% (Surveillance, Epidemiology, and End Results [SEER], 2019).

### Oncogenic RAS and KRAS G12C Mutation

Several proto-oncogene mutations have been implicated in the development of NSCLC. Among these, mutations in the *RAS* family of proto-oncogenes are among the most prevalent. The *RAS* family of proto-oncogenes consists of 3 closely related genes that encode guanosine triphosphatases (GTPases) responsible for regulating cellular proliferation and survival (Simanshu et al, 2017; Barbacid, 1987). Different tumor types are associated with mutations in certain isoforms of *RAS,* with Kirsten rat sarcoma viral oncogene homolog (KRAS) being the most frequently mutated isoform in most cancers (Prior et al, 2012).

Of the *KRAS* mutations, an estimated 80% occur at codon 12. The *KRAS G12C* mutation in codon 12 is a single guanine to thymine substitution that results in a glycine to cysteine substitution at amino acid position 12. This structural change in the protein results in a defect in the association of guanosine triphosphatase-activating proteins (GAPs), thereby reducing the hydrolysis of guanosine triphosphate (GTP) by the KRAS protein. The resulting accumulation of active, GTP-bound KRAS leads to proliferative and survival signaling in tumor cells (Jones et al, 2017). It is estimated that the *KRAS G12C* mutation is present in approximately 13% of lung adenocarcinoma and has been identified as a putative oncogenic driver in this tumor type (AACR Project GENIE Consortium, 2017; Biernacka et al, 2016; Fernández-Medarde and Santos, 2011).

The role of *KRAS* mutations in human cancers, including NSCLC, has been known for decades, but no inhibitors specifically targeting *KRAS G12C* mutations have been successfully developed until recently (McCormick, 2019).

### Current Treatments and Unmet Medical Need

Treatment of advanced NSCLC has unequivocally improved since the discovery of immunotherapies (the checkpoint inhibitors) and targeted therapies for a variety of oncogenic mutations. The National Comprehensive Cancer Network (NCCN) and European Society for Medical Oncology (ESMO) treatment guidelines call for testing of all subject with NSCLC for oncogenic driver mutations (Ettinger et al, 2019; Planchard et al, 2018). However, no anticancer therapies are currently approved for the treatment of subjects with NSCLC that specifically target tumors that have the *KRAS G12C* mutation (Román et al, 2018; McCormick, 2016). Further, Oncogenic *KRAS* mutations rarely occur concomitantly with other oncogenic mutations such as the epidermal growth factor receptor gene (*EGFR*), anaplastic lymphoma kinase gene (*ALK*), B-raf gene (*BRAF*); ROS proto-oncogene 1 (*ROS1*), or neurotrophic tyrosine kinase gene (*NTRK*) (Scheffler et al, 2019; Martorell et al, 2017; Gainor et al, 2013). Thus, most subjects with oncogenic *KRAS* mutations, including the *KRAS G12C* mutation, are not candidates for currently approved targeted therapies and consequently are typically treated as subjects without targetable mutations (*i.e*., with chemotherapy, immunotherapy, or antiangiogenic agents) (Planchard et al, 2018; Van Cutsem et al, 2014).

### Unmet Medical Need

Checkpoint inhibitors have emerged as the new frontline therapy, and chemotherapy with or without an anti-angiogenic agent is the standard of care for second line or later for subjects without actionable mutations (Ettinger et al, 2019; Planchard et al, 2018). Given the low response rates to chemotherapy regimens and poor survival outcomes of subjects with NSCLC in second-line or later treatment, new biomarker-driven anticancer therapies are needed in this subject population.

Standard-of-care outcomes for subjects with advanced/metastatic NSCLC (who are not candidates for currently approved targeted therapy) in ≥ second-line therapies, who had received first-line platinum-containing chemotherapy doublets (typically cisplatin/pemetrexed), have demonstrated objective response rates (ORRs; objective response = complete response + partial response) between 5.5% to 13% with chemotherapy (typically a taxane) and between 9.7% to 22.5% with chemotherapy plus a vascular endothelial growth factor receptor (VEGFR) inhibitor (Gridelli et al, 2018; Rittmeyer et al, 2017; Herbst et al, 2016; Borghaei et al, 2015; Herbst et al, 2007). These studies have also demonstrated progression-free survival (PFS) and overall survival (OS) of 2.8 to 4.2 months and 6 to 11.4 months, respectively, for chemotherapy alone and 4.8 to 5.4 months and 9.9 to 12.6 months, respectively, for chemotherapy with a VEGFR inhibitor. In ≥ second-line therapy settings, patients unselected for *KRAS* mutations demonstrated a median PFS of 4.0 months when treated with anti-PD-1 therapy. The median OS was 12.7 months (Herbst et al., 2016).

Additionally, the *KRAS G12C* mutation was observed in the presence of high *STK11* and *KEAP1* mutation levels. *STK11* mutations have been observed at a high rate in NSCLC and even higher in *KRAS* mutated NSCLC; this co-mutation pattern has been associated with lower OS and resistance to immune checkpoint inhibitors (Scheffler et al, 2019; Ricciuti et al, 2020; Pavan et al, 2020; Tamiya et al, 2020; An et al, 2020; Uba et al, 2020). Similarly, deletion of *KEAP1* has been associated with chemoresistance in preclinical models of NSCLC, and *KEAP1* mutations have been associated with lower OS in NSCLC, while subjects with NSCLC harboring *KEAP1* mutations are less sensitive to platinum-based treatments (Jeong et al, 2020; Tian et al, 2016; Solis et al, 2010; Arbour et al, 2018; Goeman et al., 2019).

Furthermore, while the development of checkpoint inhibitors has changed the treatment paradigm for advanced cancers, such as NSCLC, most subjects do not respond to treatment. Programmed Death Ligand 1 (PD-L1) emerged as an early biomarker to be tested in immunotherapy clinical trials. While PD-L1 testing has not delivered a biomarker with broad applicability, it has demonstrated value for certain tumor types, such as NSCLC, and remains a common immune-based biomarker in present clinical practice. (Davis et al., 2019). In fact, the US Food and Drug Administration (FDA) has linked, for example, the approval of pembrolizumab for use as a single agent in certain NSCLC settings to a PD-L1 Tumor Proportion Score (TPS) of equal or greater than 1% as determined by an FDA-approved test (Pembrolizumab Prescribing Information, revised November 2020, https://www.merck.com/product/usa/pi_circulars/k/keytruda/keytruda_pi.pdf; accessed January 2020).

With these survival outcomes and treatment challenges, current therapies are inadequate and improved treatment methods for subjects with previously treated and untreated cancers are desirable.

### SUMMARY

Provided herein are methods of treating *KRAS G12C* mutated non-small cell lung cancer in a subject in need thereof, comprising administering to the subject a total daily dose of 960 mg of a compound of Formula I 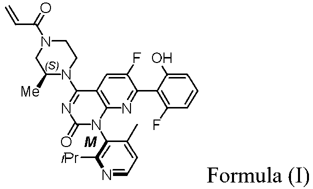 or a pharmaceutically acceptable salt thereof. In one embodiment, the subject has a PD-L1 tumor proportion score of less than 50%. In one embodiment, the subject is positive for a mutation of *STK11*, such as a loss-of-function mutation of *STK11*. In one embodiment, the subject is positive for a mutation of *KEAP1*, such as a loss-of-function mutation of *KEAP1*. In one embodiment, the subject has no brain metastasis. In one embodiment, the subject has no bone metastasis.

Further provided herein are methods of identifying a subject sensitive to the methods of treatment disclosed herein. Also provided herein are methods of determining a treatment for a subject with certain characteristics as discloses herein.

### BRIEF DESCRIPTION OF THE DRAWINGS

Figure 1 shows the objective response rate (ORR, %, non-responders/responders) for different Programmed death-ligand 1 (PD-L1) expression level groups by tumor proportion score (TPS) (<1%, 1-49% and ≥50%) in a subject population evaluable for efficacy per central review (N=124), who had available biomarker data (N=86) (data cutoff December 1, 2020).
Figure 2 shows the objective response rate (ORR, %, non-responders/responders) by *STK11*/*KEAP1* co-occurring mutations in a subject population (N=124) evaluable for efficacy per central review, who had available biomarker data (N=104) (data cutoff December 1, 2020). WT = wild type. MUT = mutant.
Figure 3 shows the objective response rate (ORR, %, non-responders/responders) by *TP53*/*STK11*/*KEAP1* (data cutoff March 15, 2021).
Figure 4 shows the objective response rate (ORR, %) by PD-L1 expression level groups by TPS (<1%, 1-49% and ≥50%) overlaid by *STK11* co-occurring mutations (data cutoff March 15, 2021). Wt = wild type. Mut = mutant.
Figure 5 shows the objective response rate (ORR, %) by PD-L1 expression level groups by TPS (<1%, 1-49% and ≥50%) overlaid by *KEAP1* co-occurring mutations (data cutoff March 15, 2021). Wt = wild type. Mut = mutant.
Figure 6 shows the objective response rate (ORR, %) by PD-L1 expression level groups by TPS (<1%, 1-49% and ≥50%) overlaid by *TP53* co-occurring mutations (data cutoff March 15, 2021). Wt = wild type. Mut = mutant.
Figure 7 shows the progression free survival (PFS) by *STK11* status (data cutoff March 15, 2021). WT = wild type. Mut = mutant.
Figure 8 shows the progression free survival (PFS) by *KEAP1* status (data cutoff March 15, 2021). WT = wild type. Mut = mutant.

### DETAILED DESCRIPTION

### First Aspect: PD-L1 Protein Expression

Provided herein as Embodiment 1 is a method of treating non-small cell lung cancer in a subject in need thereof, comprising (A) (i) assaying a sample obtained from the subject for a *KRAS G12C* mutation and (ii) assaying a sample obtained from the subject for a PD-L1 tumor proportion score, and (B) administering a total daily dose of 960 mg of a compound of Formula I 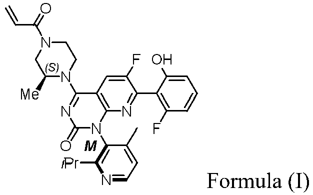 or a pharmaceutically acceptable salt thereof to a subject having non-small cell lung cancer that is positive for a *KRAS G12C* mutation and has PD-L1 tumor proportion score of less than 50%.

Provided herein as Embodiment 2 is a method of treating *KRAS G12C* mutated non-small cell lung cancer in a subject in need thereof, comprising administering to the subject a total daily dose of 960 mg of a compound of Formula I 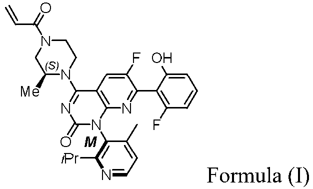 or a pharmaceutically acceptable salt thereof, wherein the subject has a PD-L1 tumor proportion score of less than 50%.

Provided herein as Embodiment 3 is a method of treating non-small cell lung cancer in a subject in need thereof, comprising administering to the subject a total daily dose of 960 mg of a compound of Formula I 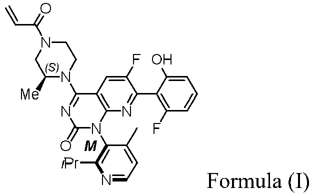 or a pharmaceutically acceptable salt thereof, wherein the subject comprises cells that are positive for a *KRAS G12C* mutation and wherein the subject comprises cells that have a PD-L1 tumor proportion score of less than 50%.

Provided herein as Embodiment 4 is a method of treating non-small cell lung cancer in a subject in need thereof, comprising administering to the subject a total daily dose of 960 mg of a compound of Formula I 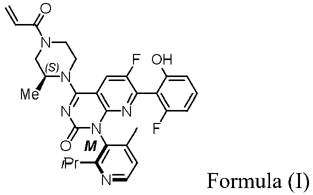 or a pharmaceutically acceptable salt thereof, wherein the non-small cell lung cancer comprises cells that are positive for a *KRAS G12C* mutation and wherein the non-small cell lung cancer comprises cells that have a PD-L1 tumor proportion score of less than 50%.

Provided herein as Embodiment 5 is a method of identifying a subject having non-small cell lung cancer as sensitive to treatment with a compound of Formula I 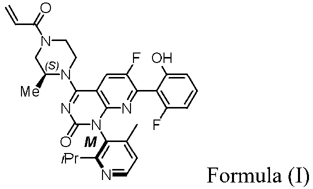 or a pharmaceutically acceptable salt thereof, comprising (i) assaying a sample obtained from the subject for a *KRAS G12C* mutation and (ii) assaying a sample obtained from the subject for a PD-L1 tumor proportion score, wherein the subject is identified as sensitive to treatment with a compound of Formula I or a pharmaceutically acceptable salt thereof, when a sample is positive for a *KRAS G12C* mutation and a sample has a PD-L1 tumor proportion score of less than 50%.

Provided herein as Embodiment 6 is a method of determining a treatment for a subject having non-small cell lung cancer, comprising (i) assaying a sample obtained from the subject for a *KRAS G12C* mutation and (ii) assaying a sample obtained from the subject for a PD-L1 tumor proportion score, wherein the treatment determined for the subject comprises administering a compound of Formula I 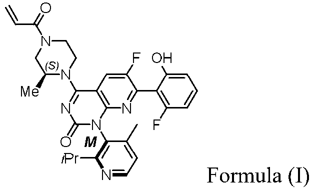 or a pharmaceutically acceptable salt thereof to the subject, when a sample is positive for a *KRAS G12C* mutation and a sample has a PD-L1 tumor proportion score of less than 50%.

Provided herein as Embodiment 7 is the method according to any one of Embodiments 1-6, wherein the PD-L1 tumor proportion score is equal or more than 1% and less than 50%.

Provided herein as Embodiment 8 is the method according to any one of Embodiments 1-6, wherein the PD-L1 tumor proportion score is less than 1%.

Provided herein as Embodiment 9 is the method according any one of Embodiments 1-8, wherein the PD-L1 tumor proportion score is determined using an immunohistochemistry (IHC) test.

Provided herein as Embodiment 10 is the method according to Embodiment 9, wherein the test is a PD-L1 IHC 22C3 pharmDx test.

### Second Aspect: STK11 and KEAP1 Co-Occurring Mutations

Provided herein as Embodiment 11 is a method of treating non-small cell lung cancer in a subject in need thereof, comprising (A) (i) assaying a sample obtained from the subject for a *KRAS G12C* mutation and (ii) assaying a sample obtained from the subject for a mutation of *STK11,* such as a loss-of-function mutation of *STK11,* and (B) administering a total daily dose of 960 mg of a compound of Formula I 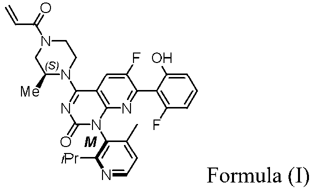 or a pharmaceutically acceptable salt thereof to a subject having non-small cell lung cancer that is positive for a *KRAS G12C* mutation and positive for a mutation of *STK11,* such as a loss-of-function mutation of *STK11.*

Provided herein as Embodiment 12 is the method according to Embodiment 11, wherein step (A) further comprises (iii) assaying a sample obtained from the subject for a mutation of *KEAP1*, such as a loss-of-function mutation of *KEAP1*, and wherein in step (B) the subject has non-small cell lung cancer that is positive for a *KRAS G12C* mutation, positive for mutation of *STK11,* such as a loss-of function mutation of *STK11,* and positive for a mutation of *KEAP1*, such as a loss-of function mutation of *KEAP1*.

Provided herein as Embodiment 13 is the method according to Embodiment 11, wherein step (A) further comprises (iii) assaying a sample obtained from the subject for a wild-type of *KEAP1* and wherein in step (B) the subject has non-small cell lung cancer that is positive for a *KRAS G12C* mutation, positive for a mutation of *STK11,* such as a loss-of function mutation of *STK11,* and positive for a wild-type of *KEAP1*.

Provided herein as Embodiment 14 is a method of treating *KRAS G12C* mutated non-small cell lung cancer in a subject in need thereof, comprising administering to the subject a total daily dose of 960 mg of a compound of Formula I 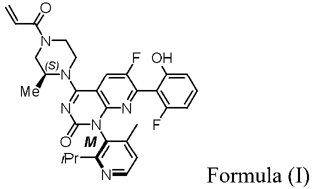 or a pharmaceutically acceptable salt thereof, wherein the subject is positive for a mutation of *STK11,* such as a loss-of-function mutation of *STK11.*

Provided herein as Embodiment 15 is the method of Embodiment 14, wherein the subject is positive for a mutation of *KEAP1*, such as a loss-of-function mutation of *KEAP1*.

Provided herein as Embodiment 16 is the method of Embodiment 14, wherein the subject is positive for a wild-type of *KEAP1*.

Provided herein as Embodiment 17 is a method of treating non-small cell lung cancer in a subject in need thereof, comprising administering to the subject a total daily dose of 960 mg of a compound of Formula I 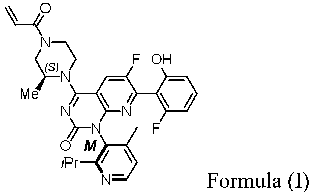 or a pharmaceutically acceptable salt thereof, wherein the subject comprises cells that are positive for a *KRAS G12C* mutation and positive for a mutation of *STK11,* such as a loss-of-function mutation of *STK11.*

Provided herein as Embodiment 18 is the method of Embodiment 17, wherein the subject further comprises cells that are positive for a wild-type of *KEAP1*.

Provided herein as Embodiment 19 is the method of Embodiment 17, wherein the subject further comprises cells that are positive for a mutation of *KEAP1*, such as a loss-of-function mutation of*KEAP1*.

Provided herein as Embodiment 20 is a method of treating non-small cell lung cancer in a subject in need thereof, comprising administering to the subject a total daily dose of 960 mg of a compound of Formula I 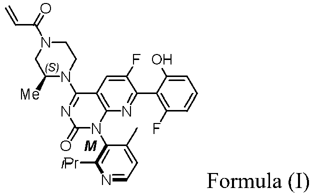 or a pharmaceutically acceptable salt thereof, wherein the non-small cell lung cancer comprises cells that are positive for a *KRAS G12C* mutation and positive for a mutation of *STK11,* such as a loss-of-function mutation of *STK11.*

Provided herein as Embodiment 21 is the method of Embodiment 20, wherein the cancer further comprises cells that are positive for a wild-type of *KEAP1*.

Provided herein as Embodiment 22 is the method of Embodiment 20, wherein the cancer further comprises cells that are positive for a mutation of *KEAP1*, such as a loss-of-function mutation of*KEAP1*.

Provided herein as Embodiment 23 is a method of identifying a subject having non-small cell lung cancer as sensitive to treatment with a compound of Formula I 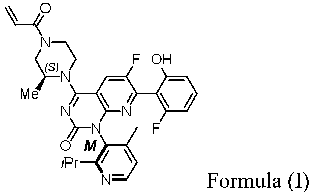 or a pharmaceutically acceptable salt thereof, comprising (i) assaying a sample obtained from the subject for a *KRAS G12C* mutation and (ii) assaying a sample obtained from the subject for a mutation of *STK11*, such as a loss-of-function mutation of *STK11,* wherein the subject is identified as sensitive to treatment with a compound of Formula I or a pharmaceutically acceptable salt thereof, when a sample is positive for a *KRAS G12C* mutation and a sample is positive for a mutation of *STK11,* such as a loss-of-function mutation of *STK11.*

Provided herein as Embodiment 24 is the method according to Embodiment 23, wherein the method further comprises (iii) assaying a sample obtained from the subject for a mutation of *KEAP1*, such as a loss-of-function mutation of *KEAP1*, wherein the subject is identified as sensitive to treatment with a compound of Formula I or a pharmaceutically acceptable salt thereof, when a sample is positive for a *KRAS G12C* mutation, a sample is positive for a mutation of *STK11,* such as a loss-of function mutation of *STK11,* and a sample is positive for mutation of *KEAP1*, such as a loss-of function mutation of*KEAP1*.

Provided herein as Embodiment 25 is the method according to Embodiment 23, wherein the method further comprises (iii) assaying a sample obtained from the subject for a wild-type of*KEAP1*, wherein the subject is identified as sensitive to treatment with a compound of Formula I or a pharmaceutically acceptable salt thereof, when a sample is positive for a *KRAS G12C* mutation, a sample is positive for a mutation of *STK11,* such as a loss-of function mutation of *STK11,* and a sample is positive for wild-type of *KEAP1*.

Provided herein as Embodiment 26 is a method of determining a treatment for a subject having non-small cell lung cancer, comprising (i) assaying a sample obtained from the subject for a *KRAS G12C* mutation and (ii) assaying a sample obtained from the subject for a mutation of *STK11,* such as a loss-of-function mutation of *STK11,* wherein the treatment determined for the subject comprises administering a compound of Formula I 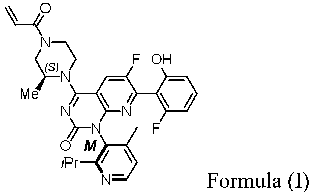 or a pharmaceutically acceptable salt thereof to the subject, when a sample is positive for a *KRAS G12C* mutation and a sample is positive for a mutation of *STK11,* such as a loss-of-function mutation of *STK11.*

Provided herein as Embodiment 27 is the method according to Embodiment 26, wherein the method further comprises (iii) assaying a sample obtained from the subject for a mutation of *KEAP1*, such as a loss-of-function mutation of *KEAP1*, wherein the treatment determined for the subject comprises administration of a compound of Formula I or a pharmaceutically acceptable salt thereof to the subject, when a sample is positive for a *KRAS G12C* mutation, a sample is positive for a mutation of *STK11*, such as a loss-of-function mutation of *STK11,* and a sample is positive for a loss-of function mutation of *KEAP1*.

Provided herein as Embodiment 28 is the method according to Embodiment 26, wherein the method further comprises (iii) assaying a sample obtained from the subject for a wild-type of*KEAP1*, wherein the treatment determined for the subject comprises administration of a compound of Formula I or a pharmaceutically acceptable salt thereof to the subject, when a sample is positive for a *KRAS G12C* mutation, a sample is positive for a mutation of *STK11,* such as a loss-of-function mutation of *STK11,* and a sample is positive for a wild-type of *KEAP1*.

Provided herein as Embodiment 29 is a method of treating non-small cell lung cancer in a subject in need thereof, comprising (A) (i) assaying a sample obtained from the subject for a *KRAS G12C* mutation and (ii) assaying a sample obtained from the subject for a mutation of *KEAP1*, such as a loss-of-function mutation of *KEAP1*, and (B) administering a total daily dose of 960 mg of a compound of Formula I 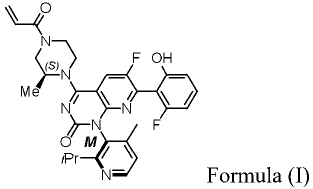 or a pharmaceutically acceptable salt thereof to a subject having non-small cell lung cancer that is positive for a *KRAS G12C* mutation and positive for a mutation of *KEAP1*, such as a loss-of-function mutation of *KEAP1*.

Provided herein as Embodiment 30 is the method according to Embodiment 29, wherein step (A) further comprises (iii) assaying a sample obtained from the subject for a mutation of *STK11,* such as a loss-of-function mutation of *STK11,* and wherein in step (B) the subject has non-small cell lung cancer that is positive for a *KRAS G12C* mutation, positive for a mutation of *KEAP1*, such as a loss-of function mutation of *KEAP1*, and positive for a mutation of *STK11*, such as a loss-of function mutation of *STK11.*

Provided herein as Embodiment 31 is the method according to Embodiment 29, wherein step (A) further comprises (iii) assaying a sample obtained from the subject for a wild-type of *KEAP1* and wherein in step (B) the subject has non-small cell lung cancer that is positive for a *KRAS G12C* mutation, positive for a mutation of *KEAP1*, such as a loss-of function mutation of *KEAP1*, and positive for a wild-type of *STK11.*

Provided herein as Embodiment 32 is a method of treating *KRAS G12C* mutated non-small cell lung cancer in a subject in need thereof, comprising administering to the subject a total daily dose of 960 mg of a compound of Formula I 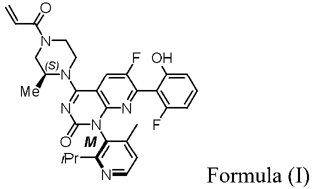 or a pharmaceutically acceptable salt thereof, wherein the subject is positive for a mutation of *KEAP1*, such as a loss-of-function mutation of *KEAP1*.

Provided herein as Embodiment 33 is the method of Embodiment 32, wherein the subject is positive for a mutation of *STK11,* such as a loss-of-function mutation of *STK11.*

Provided herein as Embodiment 34 is the method of Embodiment 32, wherein the subject is positive for a wild-type of *STK11.*

Provided herein as Embodiment 35 is a method of treating non-small cell lung cancer in a subject in need thereof, comprising administering to the subject a total daily dose of 960 mg of a compound of Formula I 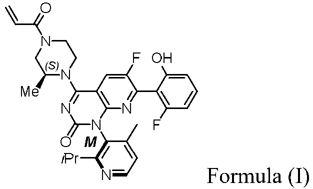 or a pharmaceutically acceptable salt thereof, wherein the subject comprises cells that are positive for a *KRAS G12C* mutation and positive for a mutation of *KEAP1*, such as a loss-of-function mutation of *KEAP1*.

Provided herein as Embodiment 36 is the method of Embodiment 35, wherein the subject further comprises cells that are positive for a wild-type of *STK11.*

Provided herein as Embodiment 37 is the method of Embodiment 35, wherein the subject further comprises cells that are positive for a mutation of *STK11,* such as a loss-of-function mutation of *STK11.*

Provided herein as Embodiment 38 is a method of treating non-small cell lung cancer in a subject in need thereof, comprising administering to the subject a total daily dose of 960 mg of a compound of Formula I 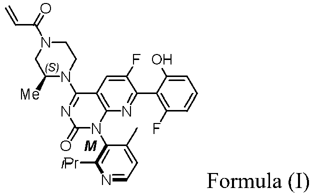 or a pharmaceutically acceptable salt thereof, wherein the non-small cell lung cancer comprises cells that are positive for a *KRAS G12C* mutation and positive for a mutation of *KEAP1*, such as a loss-of-function mutation of *KEAP1*.

Provided herein as Embodiment 39 is the method of Embodiment 38, wherein the cancer further comprises cells that are positive for a wild-type of *STK11.*

Provided herein as Embodiment 40 is the method of Embodiment 38, wherein the cancer further comprises cells that are positive for a mutation of *STK11,* such as a loss-of-function mutation of *STK11.*

Provided herein as Embodiment 41 is a method of identifying a subject having non-small cell lung cancer as sensitive to treatment with a compound of Formula I 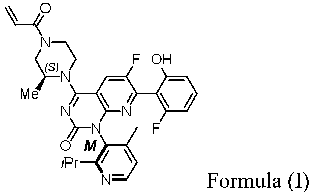 or a pharmaceutically acceptable salt thereof, comprising (i) assaying a sample obtained from the subject for a *KRAS G12C* mutation and (ii) assaying a sample obtained from the subject for a mutation of *KEAP1*, such as a loss-of-function mutation of *KEAP1*, wherein the subject is identified as sensitive to treatment with a compound of Formula I or a pharmaceutically acceptable salt thereof, when a sample is positive for a *KRAS G12C* mutation and a sample is positive for a mutation of *KEAP1*, such as a loss-of-function mutation of *KEAP1*.

Provided herein as Embodiment 42 is the method according to Embodiment 41, wherein the method further comprises (iii) assaying a sample obtained from the subject for a mutation of *STK11,* such as a loss-of-function mutation of *STK11,* wherein the subject is identified as sensitive to treatment with a compound of Formula I or a pharmaceutically acceptable salt thereof, when a sample is positive for a *KRAS G12C* mutation, a sample is positive for a mutation of *KEAP1*, such as a loss-of function mutation of *KEAP1*, and a sample is positive for a mutation of *STK11,* such as a loss-of function mutation of *STK11.*

Provided herein as Embodiment 43 is the method according to Embodiment 41, wherein the method further comprises (iii) assaying a sample obtained from the subject for a wild-type of *STK11,* wherein the subject is identified as sensitive to treatment with a compound of Formula I or a pharmaceutically acceptable salt thereof, when a sample is positive for a *KRAS G12C* mutation, a sample is positive for a mutation of *KEAP1*, such as a loss-of function mutation of *KEAP1*, and a sample is positive for wild-type of *STK11.*

Provided herein as Embodiment 44 is a method of determining a treatment for a subject having non-small cell lung cancer, comprising (i) assaying a sample obtained from the subject for a *KRAS G12C* mutation and (ii) assaying a sample obtained from the subject for a mutation of *KEAP1*, such as a loss-of-function mutation of *KEAP1*, wherein the treatment determined for the subject comprises administering a compound of Formula I 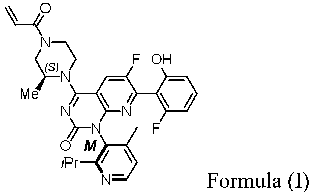 or a pharmaceutically acceptable salt thereof to the subject, when a sample is positive for a *KRAS G12C* mutation and a sample is positive for a mutation of *KEAP1*, such as a loss-of-function mutation of *KEAP1*.

Provided herein as Embodiment 45 is the method according to Embodiment 44, wherein the method further comprises (iii) assaying a sample obtained from the subject for a mutation of *STK11,* such as a loss-of-function mutation of *STK11,* wherein the treatment determined for the subject comprises administration of a compound of Formula I or a pharmaceutically acceptable salt thereof to the subject, when a sample is positive for a *KRAS G12C* mutation, a sample is positive for a mutation of *KEAP1*, such as a loss-of-function mutation of *KEAP1*, and a sample is positive for a mutation of *STK11,* such as a loss-of function mutation of *STK11.*

Provided herein as Embodiment 46 is the method according to Embodiment 44, wherein the method further comprises (iii) assaying a sample obtained from the subject for a wild-type of *STK11,* wherein the treatment determined for the subject comprises administration of a compound of Formula I or a pharmaceutically acceptable salt thereof to the subject, when a sample is positive for a *KRAS G12C* mutation, a sample is positive for a mutation of *KEAP1*, such as a loss-of-function mutation of *KEAP1*, and a sample is positive for a wild-type of *STK11.*

### Third Aspect: Absence of Brain Metastasis

Provided herein as Embodiment 47 is a method of treating non-small cell lung cancer in a subject in need thereof, comprising (A) (i) assaying a sample obtained from the subject for a *KRAS G12C* mutation and (ii) determining whether the subject has a brain metastasis, and (B) administering a total daily dose of 960 mg of a compound of Formula I 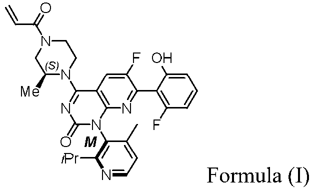 or a pharmaceutically acceptable salt thereof to a subject having non-small cell lung cancer that is positive for a *KRAS G12C* mutation and wherein the subject has no brain metastasis.

Provided herein as Embodiment 48 is a method of treating *KRAS G12C* mutated non-small cell lung cancer in a subject in need thereof, comprising administering to the subject a total daily dose of 960 mg of a compound of Formula I 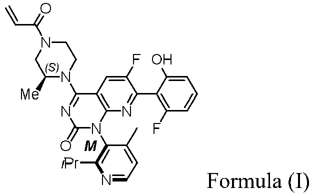 or a pharmaceutically acceptable salt thereof, wherein the subject has no brain metastasis.

Provided herein as Embodiment 49 is a method of treating non-small cell lung cancer in a subject in need thereof, comprising administering to the subject a total daily dose of 960 mg of a compound of Formula I 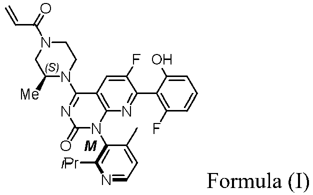 or a pharmaceutically acceptable salt thereof, wherein the subject comprises cells that are positive for a *KRAS G12C* mutation and wherein the subject has no brain metastasis.

Provided herein as Embodiment 50 is a method of treating non-small cell lung cancer in a subject in need thereof, comprising administering to the subject a total daily dose of 960 mg of a compound of Formula I 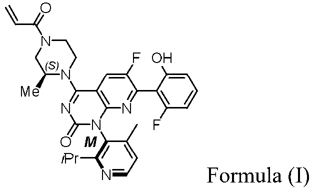 or a pharmaceutically acceptable salt thereof, wherein the non-small cell lung cancer comprises cells that are positive for a *KRAS G12C* mutation and wherein the subject has no brain metastasis.

Provided herein as Embodiment 51 is a method of identifying a subject having non-small cell lung cancer as sensitive to treatment with a compound of Formula I 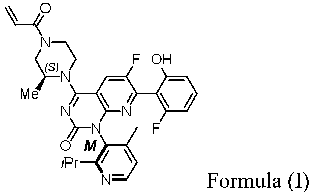 or a pharmaceutically acceptable salt thereof, comprising (i) assaying a sample obtained from the subject for a *KRAS G12C* mutation and (ii) determining whether the subject has a brain metastasis, wherein the subject is identified as sensitive to treatment with a compound of Formula I or a pharmaceutically acceptable salt thereof, when the sample is positive for a *KRAS G12C* mutation and the subject has no brain metastasis.

Provided herein as Embodiment 52 is a method of determining a treatment for a subject having non-small cell lung cancer, comprising (i) assaying a sample obtained from the subject for a *KRAS G12C* mutation and (ii) determining whether the subject has a brain metastasis, wherein the treatment determined for the subject comprises administering a compound of Formula I 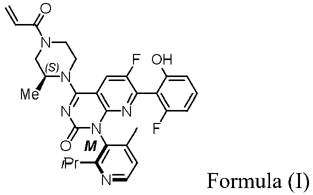 or a pharmaceutically acceptable salt thereof to the subject, when the sample is positive for a *KRAS G12C* mutation and the subject has no brain metastasis.

### Fourth Aspect: Absence of Bone Metastasis

Provided herein as Embodiment 53 is a method of treating non-small cell lung cancer in a subject in need thereof, comprising (A) (i) assaying a sample obtained from the subject for a *KRAS G12C* mutation and (ii) determining whether the subject has a bone metastasis, and (B) administering a total daily dose of 960 mg of a compound of Formula I 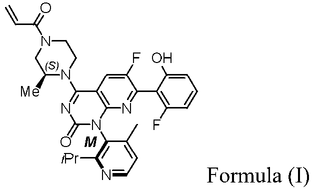 or a pharmaceutically acceptable salt thereof to a subject having non-small cell lung cancer that is positive for a *KRAS G12C* mutation and wherein the subject has no bone metastasis.

Provided herein as Embodiment 54 is a method of treating *KRAS G12C* mutated non-small cell lung cancer in a subject in need thereof, comprising administering to the subject a total daily dose of 960 mg of a compound of Formula I 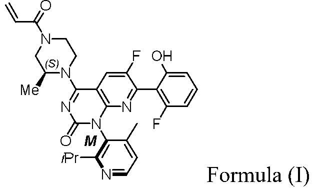 or a pharmaceutically acceptable salt thereof, wherein the subject has no bone metastasis.

Provided herein as Embodiment 55 is a method of treating non-small cell lung cancer in a subject in need thereof, comprising administering to the subject a total daily dose of 960 mg of a compound of Formula I 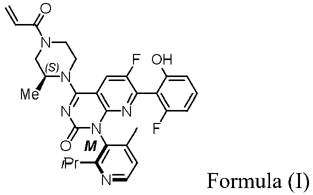 or a pharmaceutically acceptable salt thereof, wherein the subject comprises cells that are positive for a *KRAS G12C* mutation and wherein the subject has no bone metastasis.

Provided herein as Embodiment 56 is a method of treating non-small cell lung cancer in a subject in need thereof, comprising administering to the subject a total daily dose of 960 mg of a compound of Formula I 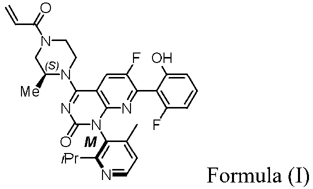 or a pharmaceutically acceptable salt thereof, wherein the non-small cell lung cancer comprises cells that are positive for a *KRAS G12C* mutation and wherein the subject has no bone metastasis.

Provided herein as Embodiment 57 is a method of identifying a subject having non-small cell lung cancer as sensitive to treatment with a compound of Formula I 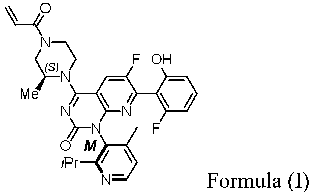 or a pharmaceutically acceptable salt thereof, comprising (i) assaying a sample obtained from the subject for a *KRAS G12C* mutation and (ii) determining whether the subject has a bone metastasis, wherein the subject is identified as sensitive to treatment with a compound of Formula I or a pharmaceutically acceptable salt thereof, when the sample is positive for a *KRAS G12C* mutation and the subject has no bone metastasis.

Provided herein as Embodiment 58 is a method of determining a treatment for a subject having non-small cell lung cancer, comprising (i) assaying a sample obtained from the subject for a *KRAS G12C* mutation and (ii) determining whether the subject has a bone metastasis, wherein the treatment determined for the subject comprises administering of a compound of Formula I 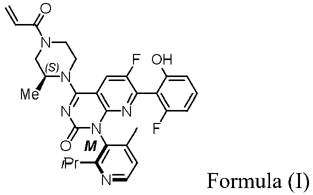 or a pharmaceutically acceptable salt thereof to the subject, when the sample is positive for a *KRAS G12C* mutation and the subject has no bone metastasis.

Provided herein as Embodiment 59 is the method according to any one of Embodiments 5, 23, 41, 51, and 57, wherein the treatment comprises administering a total daily dose of 960 mg of the compound of Formula I or a pharmaceutically acceptable salt thereof to the subject.

Provided herein as Embodiment 60 is the method according to any one of Embodiments 6, 26, 44, and 58, wherein the treatment comprises administering a total daily dose of 960 mg of the compound of Formula I or a pharmaceutically acceptable salt thereof to the subject.

### Fifth Aspect: Absence of Prior Platinum-Based Chemotherapy

Provided herein as Embodiment 61 is a method of treating non-small cell lung cancer in a subject in need thereof, comprising (A) (i) assaying a sample obtained from the subject for a *KRAS G12C* mutation and (ii) determining whether the subject has received a prior systemic platinum-based chemotherapy, and (B) administering a total daily dose of 960 mg of a compound of Formula I 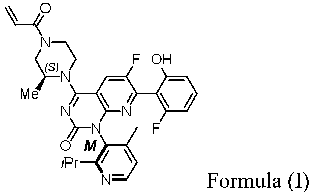 or a pharmaceutically acceptable salt thereof to a subject having non-small cell lung cancer that is positive for a *KRAS G12C* mutation and wherein the subject has not received a prior platinum-based chemotherapy.

Provided herein as Embodiment 62 is a method of treating *KRAS G12C* mutated non-small cell lung cancer in a subject in need thereof, comprising administering to the subject a total daily dose of 960 mg of a compound of Formula I 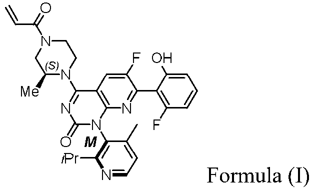 or a pharmaceutically acceptable salt thereof, wherein the subject has not received a prior systemic platinum-based chemotherapy.

Provided herein as Embodiment 63 is a method of treating non-small cell lung cancer in a subject in need thereof, comprising administering to the subject a total daily dose of 960 mg of a compound of Formula I 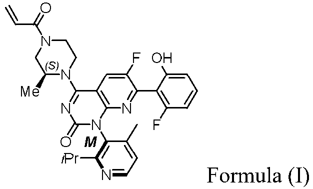 or a pharmaceutically acceptable salt thereof, wherein the subject comprises cells that are positive for a *KRAS G12C* mutation and wherein the subject has not received a prior systemic platinum-based chemotherapy.

Provided herein as Embodiment 64 is a method of treating non-small cell lung cancer in a subject in need thereof, comprising administering to the subject a total daily dose of 960 mg of a compound of Formula I 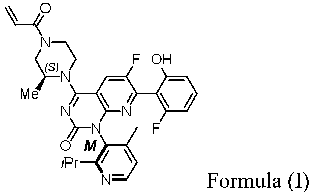 or a pharmaceutically acceptable salt thereof, wherein the non-small cell lung cancer comprises cells that are positive for a *KRAS G12C* mutation and wherein the subject has not received a prior systemic platinum-based chemotherapy.

Provided herein as Embodiment 65 is a method of identifying a subject having non-small cell lung cancer as sensitive to treatment with a compound of Formula I 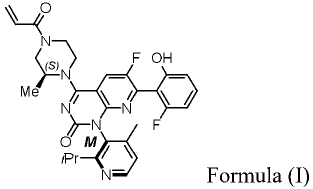 or a pharmaceutically acceptable salt thereof, comprising (i) assaying a sample obtained from the subject for a *KRAS G12C* mutation and (ii) determining whether the subject has received a prior systemic platinum-based chemotherapy, wherein the subject is identified as sensitive to treatment with a compound of Formula I or a pharmaceutically acceptable salt thereof, when the sample is positive for a *KRAS G12C* mutation and the subject has not received a prior systemic platinum-based chemotherapy.

Provided herein as Embodiment 66 is a method of determining a treatment for a subject having non-small cell lung cancer, comprising (i) assaying a sample obtained from the subject for a *KRAS G12C* mutation and (ii) determining whether the subject has received a prior systemic platinum-based chemotherapy, wherein the treatment determined for the subject comprises administration of a compound of Formula I 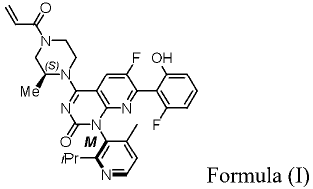 or a pharmaceutically acceptable salt thereof to the subject, when the sample is positive for a *KRAS G12C* mutation and the subject has not received a prior systemic platinum-based chemotherapy.

Provided herein as Embodiment 67 is the method according to Embodiment 65, wherein the treatment comprises administering a total daily dose of 960 mg of the compound of Formula I or a pharmaceutically acceptable salt thereof to the subject.

Provided herein as Embodiment 68 is the method according to Embodiment 66, wherein the treatment comprises administering a total daily dose of 960 mg of the compound of Formula I or a pharmaceutically acceptable salt thereof to the subject.

### Additional Embodiments of the Disclosure

Provided herein as Embodiment 69 is the method according to any one of Embodiments 61-68, wherein the subject has received at least one prior anticancer systemic therapy.

Provided herein as Embodiment 70 is the method according to any one of Embodiments 1-60, wherein the subject has received at least one prior anticancer systemic therapy.

Provided herein as Embodiment 71 is the method according to any one of Embodiments 1-70, wherein the subject has received one, two, or three prior systemic therapies.

Provided herein as Embodiment 72 is the method according to any one of Embodiments 1-70, wherein the subject has received one prior anticancer systemic therapy.

Provided herein as Embodiment 73 is the method according to any one of Embodiments 1-70, wherein the subject has received two prior anticancer systemic therapies.

Provided herein as Embodiment 74 is the method according to Embodiments 1-70, wherein the subject as received three prior anticancer systemic therapies.

Provided herein as Embodiment 75 is the method according to Embodiment 69 or Embodiment 70, wherein one prior anticancer systemic therapy is an anti-PD1 or anti-PD-L1 immunotherapy.

Provided herein as Embodiment 76 is the method according to Embodiment 70, wherein one prior anticancer systemic therapy is a platinum-based chemotherapy.

Provided herein as Embodiment 77 is the method according to any one of Embodiments 1-76, wherein the non-small cell lung cancer is locally advanced or metastatic non-small cell lung cancer.

Provided herein as Embodiment 78 is the method according to any one of Embodiments 1-77, wherein the subject has an Eastern Cooperative Oncology Group (ECOG) Performance Status of 1 or 0.

Provided herein as Embodiment 79 is the method according to any one of Embodiments 1-78, wherein the subject is 18 years or older.

Provided herein as Embodiment 80 is the method according to any one of Embodiments 1-79, wherein the compound is administered as a free base.

Provided herein as Embodiment 81 is the method according to Embodiment 80, wherein the compound is administered as a crystalline form of the free base.

Provided herein as Embodiment 82 is the method according to Embodiment 81, wherein the crystalline form is a crystalline anhydrous form.

Provided herein as Embodiment 83 is the method according to Embodiment 82, wherein the crystalline anhydrous form is crystalline anhydrous Form I.

Provided herein as Embodiment 84 is the method according to any one of Embodiments 1-83, wherein the compound is administered as a pharmaceutical composition comprising the compound and a pharmaceutically acceptable excipient.

Provided herein as Embodiment 85 is the method according to Embodiment 84, wherein the pharmaceutical composition is a solid dosage form.

Provided herein as Embodiment 86 is the method according to Embodiment 84 or embodiment 85, wherein the pharmaceutical composition is for oral administration.

Provided herein as Embodiment 87 is the method according to any one of Embodiments 84-86, wherein the pharmaceutical composition is a tablet.

Provided herein as Embodiment 88 is the method according to Embodiment 87, wherein the tablet comprises a fraction of the total daily dose of the compound of Formula I.

Provided herein as Embodiment 89 is the method according to any one of Embodiments 1-87, wherein total daily dose of the compound is administered once daily.

Provided herein as Embodiment 90 is the method according to any one of Embodiments 1-87, wherein the total daily dose of the compound is administered over two separate administrations at a dose of 480 mg each.

Provided herein as Embodiment 91 is the method according to any one of Embodiments 1-90, wherein the treatment results in inducing or increasing tumor regression in the subject.

Provided herein as Embodiment 92 is the method according to any one of Embodiments 1-90, wherein the treatment results in reducing tumor or cancer growth in the subject.

Provided herein as Embodiment 93 is the method according to any one of Embodiments 1-90, wherein the treatment results in inducing or increasing death of cancer cells in the subject.

For avoidance of doubt, it is noted that the disclosure also includes embodiments encompassing one or more embodiments of one or more aspects combined. For example, provided herein is also an embodiment that encompasses (i) the embodiments of the First Aspect of the disclosure or (ii) the embodiments of the Second Aspect of the disclosure. More specifically, the disclosure also encompasses an embodiment that recites (i) the characteristics of Embodiment 8 or (ii) the characteristics of Embodiment 14, or both. For example, the disclosure provides an embodiment, wherein the embodiment is a method of treating *KRAS G12C* mutated non-small cell lung cancer in a subject in need thereof, comprising administering to the subject a total daily dose of 960 mg of a compound of Formula I 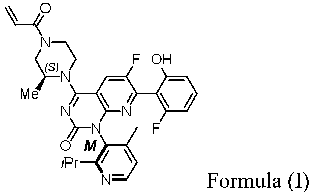 or a pharmaceutically acceptable salt thereof, (i) wherein the subject has a PD-L1 tumor proportion score of less than 1% or (ii) wherein the subject is positive for a mutation of *STK11,* such as a loss-of-function mutation of *STK11;* or both. The characteristics of the embodiments listed in the sections entitled "Additional Embodiments of the Disclosure" and "Additional Subpopulations" equally apply to any embodiments encompassing one or more embodiments of one or more aspects combined.

### Alternative Set of Embodiments of the Disclosure

Provided herein as Alternative Embodiment 1 is a method of treating *KRAS G12C* mutated non-small cell lung cancer in a subject in need thereof, comprising administering to the subject a total daily dose of 960 mg of a compound of Formula I 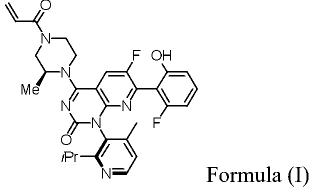 or a pharmaceutically acceptable salt thereof, wherein the subject has a PD-L1 tumor proportion score of less than 50%.

Provided herein as Alternative Embodiment 2 is the method according to Alternative Embodiment 1, wherein the PD-L1 tumor proportion score is equal or more than 1% and less than 50%.

Provided herein as Alternative Embodiment 3 is the method according to Alternative Embodiment 1, wherein the PD-L1 tumor proportion score is less than 1%.

Provided herein as Alternative Embodiment 4 is a method of treating *KRAS G12C* mutated non-small cell lung cancer in a subject in need thereof, comprising administering to the subject a total daily dose of 960 mg of a compound of Formula I 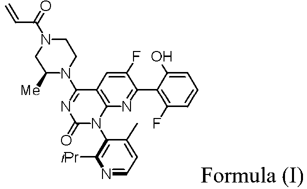 or a pharmaceutically acceptable salt thereof, wherein the subject is positive for a loss-of-function mutation of *STK11.*

Provided herein as Alternative Embodiment 5 is the method of Alternative Embodiment 4, wherein the subject is positive for a loss-of-function mutation of*KEAP1*.

Provided herein as Alternative Embodiment 6 is the method of Alternative Embodiment 4, wherein the subject is positive for a wild-type of *KEAP1*.

Provided herein as Alternative Embodiment 7 is a method of treating *KRAS G12C* mutated non-small cell lung cancer in a subject in need thereof, comprising administering to the subject a total daily dose of 960 mg of a compound of Formula I 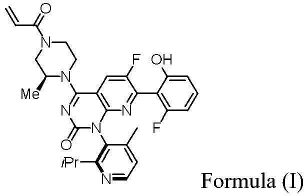 or a pharmaceutically acceptable salt thereof, wherein (i) the subject has a PD-L1 tumor proportion score of less than 50% or (ii) the subject is positive for a loss-of-function mutation of *STK11.*

Provided herein as Alternative Embodiment 8 is a method of treating *KRAS G12C* mutated non-small cell lung cancer in a subject in need thereof, comprising administering to the subject a total daily dose of 960 mg of a compound of Formula I 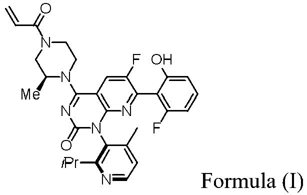 or a pharmaceutically acceptable salt thereof, wherein (i) the subject has a PD-L1 tumor proportion score of less than 50% and (ii) the subject is positive for a loss-of-function mutation of *STK11.*

Provided herein as Alternative Embodiment 9 is a method of treating *KRAS G12C* mutated non-small cell lung cancer in a subject in need thereof, comprising administering to the subject a total daily dose of 960 mg of a compound of Formula I 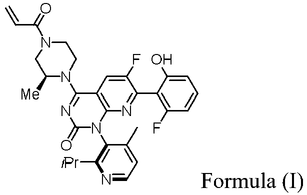 or a pharmaceutically acceptable salt thereof, wherein (i) the subject has a PD-L1 tumor proportion score of less than 50%, (ii) the subject is positive for a loss-of-function mutation of *STK11,* or (iii) the subject has a PD-L1 tumor proportion score of less than 50% and the subject is positive for a loss-of-function mutation of *STK11.*

Provided herein as Alternative Embodiment 10 is the method according to any one of Alternative Embodiments 7-9, wherein the PD-L1 tumor proportion score is equal or more than 1% and less than 50%.

Provided herein as Alternative Embodiment 11 is the method according to any one of Alternative Embodiments 7-9, wherein the PD-L1 tumor proportion score is less than 1%.

Provided herein as Alternative Embodiment 12 is the method of any one of Alternative Embodiments 7-11, wherein the subject is positive for a loss-of-function mutation of*KEAP1*.

Provided herein as Alternative Embodiment 13 is the method of any one of Alternative Embodiments 7-11, wherein the subject is positive for a wild-type of *KEAP1*.

Provided herein as Alternative Embodiment 14 is the method according to any one of Alternative Embodiments 1-13, wherein the subject has received no prior anticancer systemic therapy.

Provided herein as Alternative Embodiment 15 is the method according to any one of Alternative Embodiments 1-13, wherein the subject has received at least one prior anticancer systemic therapy.

Provided herein as Alternative Embodiment 16 is the method according to any one of Alternative Embodiments 1-13, wherein the subject has received one prior anticancer systemic therapy.

Provided herein as Alternative Embodiment 17 is the method according to any one of Alternative Embodiments 1-13, wherein the subject has received two prior anticancer systemic therapies.

Provided herein as Alternative Embodiment 18 is the method according to Alternative Embodiments 1-13, wherein the subject as received three prior anticancer systemic therapies.

Provided herein as Alternative Embodiment 19 is the method according to Alternative Embodiment 15 or Alternative Embodiment 16, wherein the one prior anticancer systemic therapy is an anti-PD1 or anti-PD-L1 immunotherapy.

Provided herein as Alternative Embodiment 20 is the method according to Alternative Embodiment 15 or Alternative Embodiment 16, wherein one prior anticancer systemic therapy is a platinum-based chemotherapy.

Provided herein as Alternative Embodiment 21 is the method according to any one of Alternative Embodiments 1-20, wherein the non-small cell lung cancer is stage IV non-small cell lung cancer.

Provided herein as Alternative Embodiment 22 is the method according to any one of Alternative Embodiments 1-20, wherein the non-small cell lung cancer is locally advanced or metastatic non-small cell lung cancer.

Provided herein as Alternative Embodiment 23 is the method according to any one of Alternative Embodiments 1-22, wherein the subject has an Eastern Cooperative Oncology Group (ECOG) Performance Status of 1 or 0.

Provided herein as Alternative Embodiment 24 is the method according to any one of Alternative Embodiments 1-23, wherein the subject is 18 years or older.

Provided herein as Alternative Embodiment 25 is the method according to any one of Alternative Embodiments 1-24, wherein the compound is administered as a free base.

Provided herein as Alternative Embodiment 26 is the method according to any one of Alternative Embodiments 1-25, wherein the compound is administered as a pharmaceutical composition comprising the compound and a pharmaceutically acceptable excipient.

Provided herein as Alternative Embodiment 27is the method according to Alternative Embodiment 26, wherein the pharmaceutical composition is a solid dosage form.

Provided herein as Alternative Embodiment 28 is the method according to Alternative Embodiment 26 or Alternative Embodiment 27, wherein the pharmaceutical composition is for oral administration.

Provided herein as Alternative Embodiment 29 is the method according to any one of Alternative Embodiments 26-28, wherein the pharmaceutical composition is a tablet.

Provided herein as Alternative Embodiment 30 is the method according to any one of Alternative Embodiments 1-29, wherein total daily dose of the compound is administered once daily.

The Alternative Embodiments 1-29 may also be combined with one or more embodiments listed in the sections entitled "Additional Embodiments of the Disclosure" and "Additional Subpopulations."

### Definitions

The following definitions are provided to assist in understanding the scope of this disclosure.

The term "anti PD1 or anti PD-L1 immunotherapy" as used herein refers to a treatment that comprises the administration of, for example, an programmed death receptor-1 (PD1) blocking antibody, such as pembrolizumab, or a programmed death ligand-1 (PD-L1) blocking antibody, such as atezolizumab.

The term "compound of Formula I" as used herein refers to the compound described in the Section entitled "The Compound of Formula I and Phase 1 Clinical Trial Results" hereinbelow. The compound of Formula I (AMG 510, sotorasib, 6-fluoro-7-(2-fluoro-6-hydroxyphenyl)-(1M)-1-[4-methyl-2-(propan-2-yl)pyridin-3-yl]-4-[(2S)-2- methyl-4-(prop-2-enoyl)piperazin-1-yl]pyrido[2,3-d]pyrimidin-2(1H)-one) is disclosed in U.S. Patent No. 10,519,146 (Example 41 (col. 411-415) and Example 41-1 (col. 469)), which Examples are specifically incorporated by reference herein in their entirety. U.S. Patent No. 10,519,146 is incorporated by reference herein it its entirety.

The term "crystalline anhydrous Form I" as used herein refers to the crystalline anhydrous Form I of the compound of Formula I as described in US 2020/00369662 A1, in particular paragraphs [0226]-[240] and [0346]-[0351] (Example 2), which are specifically incorporated by reference herein in their entirety. US 2020/00369662 A1 is incorporated by reference herein in its entirety.

In one embodiment, the compound of Formula I is characterized by a powder X-ray diffraction pattern comprising peaks at 9.0, 12.0, 12.6, and 19.0 ± 0.2 degrees 2 theta as measured by x-ray powder diffraction using an x-ray wavelength of 1.54 Å. In another embodiment, the compound of Formula I is characterized by a powder X-ray diffraction pattern comprising at least three, four, five, six, or seven peaks selected from 8.8, 9.0, 10.8, 12.0, 12.6, 12.8, 13.6, 14.2, 15.0, 15.4, 18.0, 18.6, 18.7, 19.0, 19.9, 20.0, 22.9, and 25.0 ± 0.2 degrees 2 theta as measured by x-ray powder diffraction using an x-ray wavelength of 1.54 Å. In yet another embodiment, the compound of Formula I is characterized by a powder X-ray diffraction pattern comprising peaks at 8.8, 9.0, 10.8, 12.0, 12.6, 12.8, 13.6, 14.2, 15.0, 15.4, 18.0, 18.6, 18.7, 19.0, 19.9, 20.0, 22.9, and 25.0 ± 0.2 degrees 2 theta as measured by x-ray powder diffraction using an x-ray wavelength of 1.54 Å. In one embodiment, the compound of Formula I is characterized by a differential scanning calorimetry thermogram comprising an endotherm with an onset of about 293°C. In one embodiment, the compound of Formula I is characterized by a thermogravimetric analysis thermogram comprising a weight loss of about 0.2% when heated from about 25 °C to about 275 °C. In one embodiment, the compound of Formula I is characterized by ¹³C solid state NMR (nuclear magnetic resonance) comprising at least three, four, five, six, or seven peaks selected from peaks at approximately 12, 13, 16, 21, 23, 31, 33, 38, 42, 44, 47, 50, 54, 107, 110, 111, 123, 124, 127, 128, 132, 145, 146, 150, 154, 156, 158, 160, 162, 166, 167, and 168 ppm. In another embodiment, the compound of Formula I is characterized by ¹³C solid state NMR comprising peaks at approximately 12, 13, 16, 21, 23, 31, 33, 38, 42, 44, 47, 50, 54, 107, 110, 111, 123, 124, 127, 128, 132, 145, 146, 150, 154, 156, 158, 160, 162, 166, 167, and 168 ppm. In one embodiment, the compound of Formula I is characterized by ¹⁹F solid state NMR comprising peaks at approximately -49, -60, -79, -90, -109, -120, -138, -150, -168, and - 179 ppm. The analytical characterizations described herein above were conducted as described in US 2020/00369662 A1 (see, e.g., paragraphs [0322]-[0328]).

The term "loss-of-function mutation" as used herein refers to a mutation (e.g., a substitution, deletion, truncation, or frameshift mutation) that results in expression of a mutant protein that no longer exhibits wild-type activity (e.g., reduced or eliminated wild-type biological activity or enzymatic activity), results in expression of only a fragment of the protein that no longer exhibits wild-type activity, or results in no expression of the wild-type protein. For example, a loss-of-function mutation affecting the *STK11* gene in a cell may result in the loss of expression of the STK11 protein, expression of only a fragment of the STK11 protein, or expression of the STK11 protein that exhibits diminished or no enzymatic activity (e.g., no serine/threonine kinase enzymatic activity) in the cancerous cell. Similarly, a loss-of-function mutation affecting the *KEAP1* gene in a cell may result in the loss of expression of the KEAP1 protein, expression of only a fragment of the KEAP1 protein, or expression of a KEAP 1 protein that exhibits diminished or no activity (e.g., inability to interact with or activate Nuclear factor erythroid 2-related factor 2 (NRF2)) in the cell.

The term "pharmaceutically acceptable salt" as used herein refers to a salt of the compound of Formula I that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, and the like; or (2) salts formed when an acidic proton present in the parent compound either is replaced by a metal ion, for example, an alkali metal ion, an alkaline earth ion, or an aluminum ion; or coordinates with an organic base such as ethanolamine, diethanolamine, triethanolamine, N-methylglucamine, dicyclohexylamine, and the like. Additional examples of such salts can be found in Berge et al., J. Pharm. Sci. 66(1):1-19 (1977). *See also* Stahl et al., Pharmaceutical Salts: Properties, Selection, and Use, 2nd Revised Edition (2011).

The term "platinum-based chemotherapy" as used herein refers to a chemotherapeutic treatment that comprises the administration of a compound comprising platinum. For example, a compound used in platinum-based chemotherapy is cisplatin or carboplatin. In a certain embodiment, cisplatin or carboplatin is used in combination with one or more compounds selected from paclitaxel (Taxol), albumin-bound paclitaxel (nab-paclitaxel, Abraxane), docetaxel (Taxotere), gemcitabine (Gemzar), vinorelbine (Navelbine), etoposide (VP-16), and pemetrexed (Alimta). Other platinum-based chemotherapies include, but are not limited to, oxaliplatin, nedaplatin, satraplatin, lobaplatin, triplatin tetranitrate, picoplatin, prolindac, and aroplatin.

The term "pharmaceutically acceptable excipient" as used herein refers to a broad range of ingredients that may be combined with a compound or salt disclosed herein to prepare a pharmaceutical composition or formulation. Typically, excipients include, but are not limited to, diluents, colorants, vehicles, anti-adherants, glidants, disintegrants, flavoring agents, coatings, binders, sweeteners, lubricants, sorbents, preservatives, and the like.

The term "subject" as used herein refers to a human subject.

### Methods of Detecting KRAS, STK11, KEAP1, and TP53 Mutation Status

Determining whether a tumor or cancer comprises *a KRAS G12C, STK11, KEAP1* or *TP53* mutation can be undertaken, for example, by assessing the nucleotide sequence encoding the KRAS, STK11, KEAP1, or TP53 protein, by assessing the amino acid sequence of the KRAS, STK11, KEAP1, or TP53 protein, or by assessing the characteristics of a putative KRAS, STK11, KEAP1, or TP53 mutant protein or any other suitable method known in the art. The nucleotide and protein sequence of wild-type human KRAS (Accession No. BC010502; available at https://www.ncbi.nlm.nih.gov/nuccore/BC010502, accessed January 2020), STK11 (Gene ID: 6794; available at https://www.ncbi.nlm.nih.gov/gene/6794; accessed January 2020), KEAP1 (Gene ID: 9817; available at https://www.ncbi.nlm.nih.gov/gene/9817; accessed January 2020), and TP53 (Gene ID: 7157; available at https://www.ncbi.nlm.nih.gov/gene/7157, accessed May 2021) are known in the art.

Methods for detecting a mutation in a *KRAS, STK11, KEAP1*, or *TP53* nucleotide sequence are known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct and/or next generation-based sequencing, primer extension reactions, electrophoresis, oligonucleotide ligation assays, hybridization assays, TaqMan assays, SNP genotyping assays, high resolution melting assays and microarray analyses. In some embodiments, samples are evaluated for a mutation, such as the *KRAS G12C* mutation, by real-time PCR. In real-time PCR, fluorescent probes specific for a certain mutation, such as the *KRAS G12C* mutation, are used. When a mutation is present, the probe binds and fluorescence is detected. In some embodiments, the mutation is identified using a direct sequencing method of specific regions (e.g., exon 2 and/or exon 3) in the *KRAS, STK11*, *KEAP1*, or *TP53* gene. This technique will identify all possible mutations in the region sequenced.

Methods for detecting a mutation in a KRAS, STK11, KEAP1, or TP53 protein are known by those of skill in the art. These methods include, but are not limited to, detection of a KRAS, STK11, KEAP1, or TP53 mutant using a binding agent (e.g., an antibody) specific for the mutant protein, protein electrophoresis and Western blotting, and direct peptide sequencing.

Methods for determining whether a tumor or cancer comprises a *KRAS G12C, STK11, KEAP1*, or *TP53* mutation can use a variety of samples. In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded (FFPE) sample. In some embodiments, the sample is a circulating cell-free DNA and/or circulating tumor cell (CTC) sample. In some embodiments, the sample is processed to a cell lysate. In some embodiments, the sample is processed to DNA or RNA. In a certain embodiment, the sample is acquired by resection, core needle biopsy (CNB), fine needle aspiration (FNA), collection of urine, or collection of hair follicles. In one embodiment, a liquid biopsy test using whole blood or cerebral spinal fluid may be used to assess *KRAS* mutation status.

In one embodiment, a test approved by a regulatory authority, such as the US Food and Drug Administration (FDA), is used to determine whether the subject has a *KRAS G12C* mutated non-small cell lung cancer or whether the tumor or tissue sample obtained from such subject contains cells with a KRAS G12C mutation. In one embodiment, the test is therascreen^{®} KRAS RGQ PCR Kit (Qiagen). The therascreen^{®} KRAS RGQ PCR Kit is a real-time qualitative PCR assay for the detection of 7 somatic mutations in codons 12 and 13 of the human *KRAS* oncogene (G12A, G12D, G12R, G12C, G12S, G12V, and G13D) using the Rotor-Gene Q MDx 5plex HRM instrument. The kit is intended for use with DNA extracted from FFPE samples of NSCLC samples acquired by resection, CNB, or FNA.

*STK11* and *KEAP1* mutation testing can be conducted with commercially available tests, such as the Resolution Bioscience Resolution ctDx Lung^{™} assay that includes 24 genes (including those actionable in NSCLC). Tissue samples may be tested using Tempus xT 648 panel.

*TP53* mutation testing can be conducted with commercially available tests, such as Foundation One, Tempus xT, Illumina TSO500, Guardant 360, or Guardant Omni.

In one embodiment the cancer has been identified the cancer has been identified as having a *KRAS G12C* mutation. In one embodiment, the cancer has been identified as having mutation of *STK11,* such as a loss-of-function mutation of *STK11.* In one embodiment, the cancer has been identified as having a mutation of *KEAP1*, such as a loss-of-function mutation of *KEAP1*. In one embodiment, the cancer has been identified as having a wild-type *STK11.* In one embodiment, the cancer has been identified as having a wild-type of *KEAP1*.

In one embodiment, the cancer has been identified as having mutation of *STK11,* such as a loss-of-function mutation of *STK11* and a wild-type *KEAP1*. In one embodiment, the cancer has been identified as having mutation of *STK11,* such as a loss-of-function mutation of *STK11* and a mutation of *KEAP1*, such as a loss-of-function mutation of *KEAP1*. In one embodiment, the cancer has been identified as having a wild-type of *STK11* and a wild-type *KEAP1*. In one embodiment, the cancer has been identified as having a wild type of *STK11* and a mutation of *KEAP1*, such as a loss-of-function mutation of *KEAP1*.

### Methods of Detecting PD-L1 Protein Expression

PD-L1 expression can be determined by methods known in the art. For example, PD-L1 expression can be detected using PD-L1 IHC 22C3 pharmDx, the FDA-approved in vitro diagnostic immunohistochemistry (IHC) test developed by Dako and Bristol-Meyers Squibb as a companion test for treatment with pembrolizumab. This is qualitative assay using Monoclonal Mouse Anti-PD-L1, Clone 22C3 PD-L1 and EnVision FLEX visualization system on Autostainer Lin 48 to detect PD-L1 in FFPE samples, such as human non-small cell lung cancer tissue. Expression levels can be measured using the tumor proportion score (TPS), which measures the percentage of viable tumor cells showing partial or complete membrane staining at any intensity. Staining can show PD-L1 expression from 0% to 100%.

PD-L1 expression can also be detected using PD-L1 IHC 28-8 pharmDx, the FDA- approved in vitro diagnostic immunohistochemistry (IHC) test developed by Dako and Merck as a companion test for treatment with nivolumab. This qualitative assay uses the Monoclonal rabbit anti-PD-L1, Clone 28-8 and EnVision FLEX visualization system on Autostainer Lin 48 to detect PD-L1 in formalin-fixed, paraffin-embedded (FFPE) human non-small cell lung cancer tissue.

Other commercially available tests for PD-L1 detection include the Ventana SP263 assay (developed by Ventana in collaboration with AstraZeneca) that utilizes monoclonal rabbit anti- PD-L1, Clone SP263 and the Ventana SP142 Assay (developed by Ventana in collaboration with Genentech/Roche) that uses rabbit monoclonal anti-PD-L1 clone SP142. Determination of PD-L1 status is indication-specific, and evaluation is based on either the proportion of tumor area occupied by PD-L1 expressing tumor-infiltrating immune cells (% IC) of any intensity or the percentage of PD-L1 expressing tumor cells (% TC) of any intensity. See, e.g., Atezolizumab Prescribing Information, revised April 2021 (available at https://www.gene.com/download/pdf/tecentriq_prescribing.pdf, accessed May 2021).

In one embodiment, a test approved by a regulatory authority, such as the US Food and Drug Administration (FDA), is used to determine the PD-L1 TPS. In another embodiment, the PD-L1 TPS is determined using an immunohistochemistry (IHC) test. In a certain embodiment, the IHC test is the PD-L1 IHC 22C3 pharmDx test. In one embodiment, the IHC test conducted with samples acquired by, for example, resection, CNB, or FNA.

In one embodiment, the subject has a PD-L1 TPS of less than 100%, 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In one embodiment the subject has a PD-L1 TPS of less than 50%, or less than 1%. In one embodiment the subject has a PD-L1 TPS of more than or equal to 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In one embodiment, the subject has a PD-L1 TPS of less than or equal to 100%, 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In one embodiment the subject has a PD-L1 TPS of less than or equal to 50%, or less than or equal to 1%. In one embodiment the subject has a PD-L1 TPS of more than 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In one embodiment, the subject has a PD-L1 TPS score a range bound by any of the values cited in the foregoing embodiments. For example, the subject has a PD-L1 TPS score in the range of less than 50% and more than or equal to 1%, less than or equal to 50% and more than 1%, less than or equal to 50% and more than or equal to 1%, or less than 50% and more than 1%.

In one embodiment, the subject has a PD-L1 TPS score in the range of less than 50% and more than or equal to 1%. In another embodiment, the subject has a PD-L1 TPS score in the range of more than or equal to 0% and less than 1%. In yet another embodiment, the subject has a PD-L1 TPS score in the range of more than 50% and less than or equal to 100%.

### Methods of Detecting Brain and Bone Metastasis

Methods to determine whether a subject has a bone or brain metastasis are known in the art.

In one embodiment brain metastasis may be diagnosed, for example, using Computerized Axial Tomography (CAT) Scan or Computerized Tomography (CT) with or without intravenous contrast agent and Magnetic Resonance Imaging (MRI) with or without intravenous contrast agent. In one embodiment, the subject has an MRI of the brain performed within 28 days prior to treatment start. If MRI is contraindicated, then a CT with contrast agent is conducted.

In one embodiment bone metastasis may be diagnosed, for example, using any one of X-ray, bone scan (bone scintigraphy), CT, MRI, Positron Emission Tomography (PET), and biopsy.

### Methods of Detecting Tumor Mutational Burden

Methods to determine the tumor mutational burden (TMB) in a subject are known in the art. The TMB can be measured by a laboratory test that uses next-generation sequencing of tumor tissue, which looks broadly for a wide range of mutations. Although not as established as measuring TMB from a biopsy sample of tumor tissue, studies are now evaluating measuring TMB from circulating tumor DNA in the plasma, making it potentially possible to test TMB from blood in the future. The TMB is reported as the number of mutations seen in a section of DNA and reported as mutations per megabase (mut/Mb). Cancers with a TMB of 10 mut/Mb or greater (called TMB-high) may be more likely to respond to drugs called immune checkpoint inhibitors that help activate the immune system to better recognize cancer cells (Fusco et al, 2021). In one embodiment TMB test used is a commercially available test, such as Foundation One, Tempus xT, Guardant Omni, and Illumina TSO500.

### Subpopulations

Further provided herein are certain methods for treating a subject in need thereof, wherein the subject has certain characteristics in addition to the status regarding KRAS, STK11, KEAP1, TP53, PD-L1 TPS, or bone or brain metastases discussed herein above, or combinations thereof.

Also provided herein are methods of treating *KRAS G12C* mutated non-small cell lung cancer in a subject in need thereof as disclosed in the various aspects of the invention above, wherein the subject has the following characteristics:

| Characteristic A | Characteristic B | A or B | A and B | A or B, or both |
|---|---|---|---|---|
| PD-L1 TPS is < 50% | | | | |
| PD-L1 TPS is < 50% | positive for a wild-type of *STK11* | x | x | x |
| PD-L1 TPS is < 50% | positive for an *STK11* mutant | x | x | x |
| PD-L1 TPS is < 50% | positive for a loss of function mutation of *STK11* | x | x | x |
| PD-L1 TPS is < 50% | positive for a wild-type of *KEAP1* | x | x | x |
| PD-L1 TPS is < 50% | positive for an *KEAP1* mutant | x | x | x |
| PD-L1 TPS is < 50% | positive for a loss of function mutation of *KEAP1* | x | x | x |
| PD-L1 TPS is < 50% | positive for a wild-type of *TP53* | x | x | x |
| PD-L1 TPS is < 50% | positive for a *TP53* mutant | x | x | x |
| PD-L1 TPS is < 50% | positive for a loss of function mutation of *TP53* | x | x | x |
| PD-L1 TPS is ≥ 1% and < 50%, such as 1-49% | | | | |
| PD-L1 TPS is ≥ 1% and < 50%, such as 1-49% | positive for a wild-type of *STK11* | x | x | x |
| PD-L1 TPS is ≥ 1% and < 50%, such as 1-49% | positive for an *STK11* mutant | x | x | x |
| PD-L1 TPS is ≥ 1% and < 50%, such as 1-49% | positive for a loss of function mutation of *STK11* | x | x | x |
| PD-L1 TPS is ≥ 1% and < 50%, such as 1-49% | positive for a wild-type of *KEAP1* | x | x | x |
| PD-L1 TPS is ≥ 1% and < 50%, such as 1-49% | positive for a *KEAP1* mutant | x | x | x |
| PD-L1 TPS is ≥ 1% and < 50%, such as 1-49% | positive for a loss of function mutation of *KEAP1* | x | x | x |
| PD-L1 TPS is ≥ 1% and < 50%, such as 1-49% | positive for a wild-type of *TP53* | x | x | x |
| PD-L1 TPS is ≥ 1% and < 50%, such as 1-49% | positive for a *TP53* mutant | x | x | x |
| PD-L1 TPS is ≥ 1% and < 50%, such as 1-49% | positive for a loss of function mutation of *TP53* | x | x | x |
| PD-L1 TPS is < 1% | | | | |
| PD-L1 TPS is < 1% | positive for a wild-type of *STK11* | x | x | x |
| PD-L1 TPS is < 1% | positive for an *STK11*mutant | x | x | x |
| PD-L1 TPS is < 1% | positive for a loss of function mutation of *STK11* | x | x | x |
| PD-L1 TPS is < 1% | positive for a wild-type of *KEAP1* | x | x | x |
| PD-L1 TPS is < 1% | positive for a *KEAP1* mutant | x | x | x |
| PD-L1 TPS is < 1% | positive for a loss of function mutation of *KEAP1* | x | x | x |
| PD-L1 TPS is < 1% | positive for a wild-type of *TP53* | x | x | x |
| PD-L1 TPS is < 1% | positive for a *TP53* mutant | x | x | x |
| PD-L1 TPS is < 1% | positive for a loss of function mutation of *TP53* | x | x | x |
| positive for a wild-type of *STK11* | | | | |
| positive for a wild-type of *STK11* | positive for a wild-type of *KEAP1* | x | x | x |
| positive for a wild-type of *STK11* | positive for a *KEAP1* mutant | x | x | x |
| positive for a wild-type of *STK11* | positive for a loss of function mutation of *KEAP1* | x | x | x |
| positive for an *STK11* mutant | | | | |
| positive for an *STK11* mutant | positive for a wild-type of *KEAP1* | x | x | x |
| positive for an *STK11* mutant | positive for a *KEAP1* mutant | x | x | x |
| positive for an *STK11* mutant | positive for a loss of function mutation of *KEAP1* | x | x | x |
| positive for a loss of function mutation of *STK11* | | | | |
| positive for a loss of function mutation of *STK11* | positive for a wild-type of *KEAP1* | x | x | x |
| positive for a loss of function mutation of *STK11* | positive for a *KEAP1* mutant | x | x | x |
| positive for a loss of function mutation of *STK11* | positive for a loss of function mutation of *KEAP1* | x | x | x |
| positive for a wild-type of *KEAP1* | | | | |
| positive for a *KEAP1* mutant | | | | |
| positive for a loss of function mutation of *KEAP1* | | | | |
| positive for a wild-type of *TP53* | | | | |
| positive for a *TP53* mutant | | | | |
| positive for a loss of function mutation of *TP53* | | | | |

For example, the entry in the above table where Characteristic A reads "PD-L1 TPS is < 1%" and Characteristic B is empty represents the embodiments directed to methods of treating *KRAS G12C* mutated non-small cell lung cancer in a subject in need thereof as disclosed in the various aspects of the invention above, wherein the subject has a PD-L1 tumor proportion score of less than 1%.

As a further example, the entry in the above table where Characteristic A reads "PD-L1 TPS is < 1%" and Characteristic B reads "positive for a loss of function mutation of *STK11*" represents the embodiments directed to methods of treating *KRAS G12C* mutated non-small cell lung cancer in a subject in need thereof as disclosed in the various aspects of the invention above, wherein (i) the subject has a PD-L1 tumor proportion score of less than 1% or (ii) the subject is positive for a loss-of-function mutation of *STK11* (A or B). The same entry also represents the embodiments directed to methods of treating *KRAS G12C* mutated non-small cell lung cancer in a subject in need thereof as disclosed in the various aspects of the invention above, wherein (i) the subject has a PD-L1 tumor proportion score of less than 1% and (ii) the subject is positive for a loss-of-function mutation of *STK11* (A and B). The same entry also represents the embodiments directed to methods of treating *KRAS G12C* mutated non-small cell lung cancer in a subject in need thereof as disclosed in the various aspects of the invention above, wherein (i) the subject has a PD-L1 tumor proportion score of less than 1%, (ii) the subject is positive for a loss-of-function mutation of *STK11,* or (iii) the subject has a PD-L1 tumor proportion score of less than 1% and the subject is positive for a loss-of-function mutation of *STK11* (A or B or both).

The following embodiments disclose certain additional characteristics of said subject. In one embodiment the subject has 1, 2, 3, 4, 5, 6, 7, 8, 9, or 10 or all of the following additional characteristics. In a certain embodiment the subject has one of the following additional characteristics. In a certain embodiment the subject has two of the following additional characteristics. In a certain embodiment the subject has three of the following additional characteristics. In a certain embodiment the subject has five of the following additional characteristics. In a certain embodiment the subject has seven of the following additional characteristics. In a certain embodiment the subject has ten of the following additional characteristics. In a certain embodiment the subject has all of the following additional characteristics.

In one embodiment the subject is a man. In another embodiment, the subject is a woman.

In one embodiment the subject is 18 years or older.

In one embodiment the subject has a pathologically documented, locally-advanced or metastatic malignancy with *KRAS G12C* mutation identified through molecular testing. In a certain embodiment the mutation was confirmed by central testing prior to treatment start.

In one embodiment, the subject has progressed after receiving anti-PD-1 or anti-PD-L1 immunotherapy (unless contraindicated) AND/OR platinum-based combination chemotherapy AND targeted therapy if actionable oncogenic driver mutations were identified (i.e., EGFR, ALK, and ROS1).

In one embodiment, the subject has not received more than 3 prior lines of therapy.

In one embodiment an archived tumor tissue sample (formalin fixed, paraffin embedded [FFPE] sample collected within 5 years) is available, which was obtained from the subject. In a certain embodiment, a pretreatment tumor biopsy sample is available, which was obtained from the subject. In another embodiment, an additional biopsy sample taken from the subject at the time of tumor progression is available, provided the subject has lesions that can be feasibly biopsied.

In one embodiment, the subject has a measurable disease per Response Evaluation Criteria in Solid Tumors 1.1 criteria (RECIST 1.1; Eisenhauer et al, 2009).

In one embodiment the subject has an Eastern Cooperative Oncology Group (ECOG) Performance Status of ≤ 1 (Oken et al., 1982). In a certain embodiment the subject has an ECOG Performance Status of 0. In a certain embodiment the subject has an ECOG Performance Status of 1.

In one embodiment the subject has a life expectancy of > 3 months.

In one embodiment the subject has the ability to take oral medications.

In one embodiment the subject has a QTc ≤ 470 msec (based on average of screening triplicates).

In one embodiment the subject has one, two or three of the following hematological laboratory assessments: an absolute neutrophil count (ANC) ≥ 1.5 x 10⁹/L, a platelet count ≥ 75 x 10⁹/L, a hemoglobin ≥ 9 g/dL (90 g/L).

In one embodiment the subject has an estimated glomerular filtration rate based on MDRD (Modification of Diet in Renal Disease) calculation ≥ 60 ml/min/1.73 m².

In one embodiment the subject has one, two, or three of the following hepatic laboratory assessments: aspartate aminotransferase (AST) < 2.5 x upper limit of normal (ULN) (if liver metastases are present, ≤ 5 x ULN), alanine aminotransferase (ALT) < 2.5 x ULN (if liver metastases are present, ≤ 5 x ULN), total bilirubin < 1.5 x ULN (< 2.0 x ULN for subjects with documented Gilbert's syndrome or < 3.0 x ULN for subjects for whom the indirect bilirubin level suggests an extrahepatic source of elevation).

In one embodiment the subject has a prothrombin time (PT) or partial thromboplastin time (PTT) < 1.5 x ULN, OR International normalized ratio (INR) < 1.5 or within target range if on prophylactic anticoagulation therapy.

In another embodiment the subject has no active brain metastases from non-brain tumors. In a certain embodiment, a subject who has had brain metastases resected or has received radiation therapy ending at least 4 weeks prior to treatment start is eligible for treatment if the subject meets all of the following criteria: residual neurological symptoms grade of less than 2; on stable doses of dexamethasone, if applicable; and follow-up MRI performed within 30 days showed no new lesions appearing.

In another embodiment the subject has no history or presence of hematological malignancies unless curatively treated with no evidence of disease ≥ 2 years.

In another embodiment the subject has no myocardial infarction within 6 months of the start of treatment, symptomatic congestive heart failure (New York Heart Association > class II), unstable angina, or cardiac arrhythmia requiring medication.

In another embodiment the subject has no gastrointestinal (GI) tract disease causing the inability to take oral medication, malabsorption syndrome, requirement for intravenous alimentation, uncontrolled inflammatory GI disease (e.g., Crohn's disease, ulcerative colitis).

In another embodiment the subject as no active infection requiring IV antibiotics within 1 week of start of treatment.

In another embodiment the subject has no hepatitis infection. In a certain embodiment the absence of a hepatitis infection in the subject is determined based on the following results: positive Hepatitis B Surface Antigen (HepBsAg) (indicative of chronic Hepatitis B or recent acute hepatitis B); negative HepBsAg with a positive for hepatitis B core antibody (Hepatitis B core antibody testing is not required for screening, however if this was done and was positive, then hepatitis B surface antibody [Anti-HBs] testing is necessary); undetectable anti-HBs in this setting would suggest unclear and possible infection, and needs exclusion); positive Hepatitis C virus antibody: Hepatitis C virus RNA by PCR is necessary - detectable Hepatitis C virus RNA suggests chronic hepatitis C.

In another embodiment the subject has no known positive test for HIV. In a certain embodiment, the subject is HIV negative.

In another embodiment the subject has no unresolved toxicities from prior anti-tumor therapy, defined as not having resolved to CTCAE version 5.0 grade 0 or 1.

In another embodiment the subject is not subject to anti-tumor therapy (chemotherapy, antibody therapy, molecular targeted therapy, retinoid therapy, hormonal therapy [except for a subject with breast cancer], or other investigational agent) within 28 days of treatment start. In a certain embodiment, the subject concurrently is subjected to hormone deprivation therapy for hormone-refractory prostate cancer or breast cancer.

In another embodiment the subject does not have any radiotherapy related toxicity.

In another embodiment the subject has not had major surgery within 28 days of treatment start.

In another embodiment the subject is not a woman with a positive pregnancy test.

In another embodiment the subject has not been administered a known cytochrome P450 (CYP) 3A4 sensitive substrates (with a narrow therapeutic window), within 14 days or 5 half-lives of the drug or its major active metabolite, whichever is longer, prior to treatment start.

In another embodiment the subject has not been administered a strong inducer of CYP3A4 (including herbal supplements such as St. John's wort) within 14 days or 5 half-lives (whichever is longer) prior to treatment start.

In another embodiment the subject has not had another malignancy within the past 2 years before start of treatment. In a certain embodiment the following exceptions apply: malignancy treated with curative intent and with no known active disease present for ≥ 2 years before treatment start and felt to be at low risk for recurrence by the treating physician; adequately treated non-melanoma skin cancer or lentigo maligna without evidence of disease; adequately treated cervical carcinoma in situ without evidence of disease; adequately treated breast ductal carcinoma in situ without evidence of disease; prostatic intraepithelial neoplasia without evidence of prostate cancer; or adequately treated urothelial papillary non-invasive carcinoma or carcinoma in situ.

In another embodiment the subject has not been submitted to prior treatment with a KRAS^{G12C} inhibitor.

### Formulation and Route of Administration

While it may be possible to administer the compound of Formula I disclosed herein alone in the uses described, the compound administered normally will be present as an active ingredient in a pharmaceutical composition. Thus, in one embodiment, provided herein is a pharmaceutical composition comprising a compound of Formula I disclosed herein in combination with one or more pharmaceutically acceptable excipients and, if desired, other active ingredients. *See, e.g.,* Remington: The Science and Practice of Pharmacy, Volume I and Volume II, twenty-second edition, edited by Loyd V. Allen Jr., Philadelphia, PA, Pharmaceutical Press, 2012; Pharmaceutical Dosage Forms (Vol. 1-3), Liberman et al., Eds., Marcel Dekker, New York, NY, 1992; Handbook of Pharmaceutical Excipients (3rd Ed.), edited by Arthur H. Kibbe, American Pharmaceutical Association, Washington, 2000; Pharmaceutical Formulation: The Science and Technology of Dosage Forms (Drug Discovery), first edition, edited by GD Tovey, Royal Society of Chemistry, 2018.

The compound(s) disclosed herein may be administered by any suitable route in the form of a pharmaceutical composition adapted to such a route and in a dose effective for the treatment intended. The compounds and compositions presented herein may, for example, be administered orally, mucosally, topically, transdermally, rectally, pulmonarily, parentally, intranasally, intravascularly, intravenously, intraarterial, intraperitoneally, intrathecally, subcutaneously, sublingually, intramuscularly, intrasternally, vaginally or by infusion techniques, in dosage unit formulations containing conventional pharmaceutically acceptable excipients.

The pharmaceutical composition may be in the form of, for example, a tablet, chewable tablet, minitablet, caplet, pill, bead, hard capsule, soft capsule, gelatin capsule, granule, powder, lozenge, patch, cream, gel, sachet, microneedle array, syrup, flavored syrup, juice, drop, injectable solution, emulsion, microemulsion, ointment, aerosol, aqueous suspension, or oily suspension. The pharmaceutical composition is typically made in the form of a dosage unit containing a particular amount of the active ingredient.

### The Compound of Formula I and Phase 1 Clinical Trial Results

Without wishing to be bound to any particular theory, the following is noted: The compound of Formula I (AMG 510, sotorasib, U.S. Patent No. 10,519,146 (Example 41-1)) is a novel, potent, and highly selective small molecule inhibitor that covalently binds to the KRAS protein with a G12C substitution (KRAS^{G12C}) and locks it in a guanine diphosphate (GDP)-bound, inactive state (see, e.g., U.S. Patent No. 10,519,146). By doing so, the compound of Formula I specifically binds and irreversibly inhibits the KRAS^{G12C} mutant protein. The chemical name of the compound of Formula I is 6-fluoro-7-(2- fluoro-6-hydroxyphenyl)-(1*M*)-1-[4-methyl-2-(propan-2-yl)pyridin-3-yl]-4-[(2*S*)-2-methyl-4-(prop- 2-enoyl)piperazin-1-yl]pyrido[2,3-*d*]pyrimidin-2(1*H*)-one. The chemical structure is shown below.

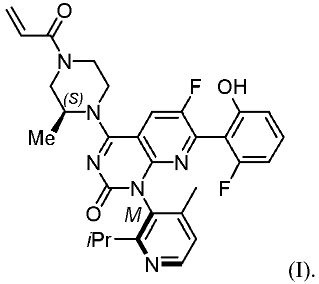

The compound of Formula I potently inhibits recombinant KRAS^{G12C} but has minimal effect on wild type KRAS or other mutant versions of KRAS. The covalent, irreversible binding and inhibition of KRAS^{G12C} by the compound of Formula I requires a reactive thiol group adjacent to the compound of Formula I binding pocket. This thiol is provided by the cysteine at KRAS position 12 (G12C), resulting in a precise interaction that is specific for KRAS^{G12C}. The inhibitor contains a thiol-reactive portion that covalently modifies the cysteine residue and locks KRAS^{G12C} in the inactive, GDP-bound conformation. This blocks the interaction of KRAS with effectors such as rapidly accelerated fibrosarcoma (RAF), thereby preventing downstream proliferation and survival signaling, including the phosphorylation of extracellular signal regulated kinase (ERK) (Canon et al, 2019; Simanshu et al, 2017; Ostrem et al, 2013; Cully and Downward, 2008). Treatment with the compound of Formula I impairs cell growth and induces apoptosis only in tumor cell lines and xenografts that have the *KRAS G12C* mutation (Canon et al, 2019). Blockade of KRAS^{G12C} signaling by the compound of Formula I also enhances antigen presentation and inflammatory cytokine production in tumors to inflame the tumor microenvironment and drive anti-tumor immunity. Thus, the compound of Formula I represents an important advance for the treatment of subjects with *KRAS G12C*-mutated tumors, such as NSCLC.

The compound of Formula I is subject of the ongoing clinical study entitled "A Phase 1/2, Open-label Study Evaluating the Safety, Tolerability, Pharmacokinetics, Pharmacodynamics, and Efficacy of AMG 510 Monotherapy in Subjects With Advanced Solid Tumors With KRAS p.G12C Mutation and AMG 510 Combination Therapy in Subjects With Advanced NSCLC With KRAS p.G12C Mutation (CodeBreak 100)" (ClinicalTrials.gov identifier: NCT03600883, available at https://www.clinicaltrials.gov/ct2/show/NCT03600883, accessed January 2020).

The primary objectives of the phase 1 portion of the study were to evaluate the safety and tolerability of the compound of Formula I and to estimate the maximum tolerated dose and/or a recommended phase 2 dose of sotorasib. For the phase 2 portion of the study, the primary objective was to evaluate the objective response rate (ORR) for the compound of Formula I as monotherapy in subjects with *KRAS* G12C-mutated advanced solid tumors. Secondary objectives for both portions of the study included other measures of the compound of Formula I efficacy (endpoints of duration of response, disease control rate, time to response, progression-free survival (PFS), and overall survival (OS)), safety, and pharmacokinetics. Subjects in phase 1 were treated with the compound of Formula I as monotherapy at 180, 360, 720, and 960 mg once daily (QD).

Certain results of the Phase 1 cohort of the CodeBreaK 100 trial (NCT03600883) have been published (Hong et al, 2020). Hong et al. discusses, *inter alia,* a set of 59 subjects with NSCLC, of which 53 (89.8%) had received prior anti-programmed cell death protein 1 (PD-1) or anti-programmed death ligand 1 (PD-L1) therapies, and all 59 subjects had received prior platinum-based chemotherapy. Briefly, the compound of Formula I was well tolerated and demonstrated a confirmed objective response (complete or partial response) rate of 32.2% across all doses tested, a disease control rate (objective response or stable disease) of 88.1 %, a median duration of response (DoR) of 10.9 months, and a median progression-free survival (mPFS) of 6.3 month in subjects with heavily pretreated NSCLC.

The recommended dose for phase 2 identified based on the results obtained in phase 1 was 960 mg QD of compound of Formula I as monotherapy.

### EXAMPLES

### EXAMPLE 1: Phase 2 Study Design

This Example presents certain results of phase 2 portion of the CodeBreaK 100 Trial (ClinicalTrials.gov identifier: NCT03600883). Data cutoff for the analysis presented herein was September 1, 2020, unless indicated otherwise.

As discussed above, in phase 2 of the CodeBreaK 100 Trial (ClinicalTrials.gov identifier: NCT03600883) subjects with locally advanced or metastatic *KRAS G12C* mutant NSCLC were treated with the compound of Formula I as monotherapy at 960 mg QD. Subjects were to continue sotorasib treatment until disease progression (unless the subject is eligible for continued treatment), treatment intolerance, withdrawal of consent, death, or other protocol-defined reasons.

Subjects were admitted to the phase 2 portion of the trial based on the following inclusion and exclusion criteria.

### Inclusion Criteria

- Subject has provided informed consent prior to initiation of any study specific activities/procedures.
- Men or women: 18 years of age or older.
- Pathologically documented, locally-advanced or metastatic malignancy with *KRAS G12C* mutation identified through molecular testing. For phase 2, the mutation was confirmed by central testing prior to enrollment.
- Subjects must have progressed after receiving anti-PD-1 or anti-PD-L1 immunotherapy (unless contraindicated) AND/OR platinum-based combination chemotherapy AND targeted therapy if actionable oncogenic driver mutations were identified (i.e., EGFR, ALK, and ROS1). Subjects must have received no more than 3 prior lines of therapy. The following guidance was used:
   ∘ Adjuvant therapy was counted as a line of therapy if the subject progressed on or within 6 months of adjuvant therapy administration.
   ∘ In locally advanced and unresectable NSCLC, disease progression on or within six months of end of prior curatively intended multimodal therapy was counted as a line of therapy. If chemoradiation is followed by planned systemic therapy without documented progression between chemoradiation and systemic therapy, the entire treatment course was counted as 1 line of therapy.
   ∘ Maintenance therapy following platinum doublet-based chemotherapy was not considered as a separate line of therapy.
- Subjects willing to provide archived tumor tissue samples (formalin fixed, paraffin embedded [FFPE] sample collected within 5 years) or willing to undergo pretreatment tumor biopsy.
- Subjects who have lesions that can be feasibly biopsied were asked to undergo an optional biopsy at the time of tumor progression.
- Measurable disease per Response Evaluation Criteria in Solid Tumors 1.1 criteria (RECIST 1.1; Eisenhauer et al, 2009).
- Eastern Cooperative Oncology Group (ECOG) Performance Status of ≤ 1 (Oken et al., 1982).
- Life expectancy of > 3 months, in the opinion of the investigator.
- Ability to take oral medications and willing to record daily adherence to investigational product utilizing a sponsor-provided dosing diary.
- QTc ≤ 470 msec (based on average of screening triplicates).
- Adequate hematological laboratory assessments, as follows:
   ∘ Absolute neutrophil count (ANC) ≥ 1.5 x 10⁹/L.
   ∘ Platelet count ≥ 75 x 10⁹/L.
   ∘ Hemoglobin ≥ 9 g/dL (90 g/L).
- Adequate renal laboratory assessments, as follows:
   ∘ Estimated glomerular filtration rate based on MDRD (Modification of Diet in Renal Disease) calculation ≥ 60 ml/min/1.73 m².
- Adequate hepatic laboratory assessments, as follows:
   ∘ Aspartate aminotransferase (AST) < 2.5 x upper limit of normal (ULN) (if liver metastases are present, ≤ 5 x ULN).
   ∘ Alanine aminotransferase (ALT) < 2.5 x ULN (if liver metastases are present, ≤ 5 x ULN).
   ∘ Total bilirubin < 1.5 x ULN (< 2.0 x ULN for subjects with documented Gilbert's syndrome or < 3.0 x ULN for subjects for whom the indirect bilirubin level suggests an extrahepatic source of elevation).
- Adequate coagulation laboratory assessments, as follows:
   ∘ Prothrombin time (PT) or partial thromboplastin time (PTT) < 1.5 x ULN, OR International normalized ratio (INR) < 1.5 or within target range if on prophylactic anticoagulation therapy.

### Exclusion Criteria

- Active brain metastases from non-brain tumors. Subjects who have had brain metastases resected or have received radiation therapy ending at least 4 weeks prior to study day 1 were eligible if they meet all of the following criteria:
   ∘ residual neurological symptoms grade of less than 2;
   ∘ on stable doses of dexamethasone, if applicable; and
   ∘ follow-up MRI performed within 30 days showed no new lesions appearing.
- History or presence of hematological malignancies unless curatively treated with no evidence of disease ≥ 2 years.
- Myocardial infarction within 6 months of study day 1, symptomatic congestive heart failure (New York Heart Association > class II), unstable angina, or cardiac arrhythmia requiring medication.
- Gastrointestinal (GI) tract disease causing the inability to take oral medication, malabsorption syndrome, requirement for intravenous alimentation, uncontrolled inflammatory GI disease (e.g., Crohn's disease, ulcerative colitis).
- Active infection requiring IV antibiotics within 1 weeks of study enrollment (day 1).
- Exclusion of hepatitis infection based on the following results and/or criteria:
   ∘ Positive Hepatitis B Surface Antigen (HepBsAg) (indicative of chronic Hepatitis B or recent acute hepatitis B).
   ∘ Negative HepBsAg with a positive for hepatitis B core antibody (Hepatitis B core antibody testing was not required for screening, however if this was done and was positive, then hepatitis B surface antibody [Anti-HBs] testing was necessary. Undetectable anti-HBs in this setting would suggest unclear and possible infection and needs exclusion).
   ∘ Positive Hepatitis C virus antibody: Hepatitis C virus RNA by PCR was necessary. Detectable Hepatitis C virus RNA suggests chronic hepatitis C.
- Known positive test for HIV.
- Unresolved toxicities from prior anti-tumor therapy, defined as not having resolved to CTCAE version 5.0 grade 0 or 1, or to levels dictated in the eligibility criteria with the exception of alopecia (grade 2 or 3 toxicities from prior anti-tumor therapy that are considered irreversible [defined as having been present and stable for > 6 months], such as ifosfamide related proteinuria, was allowed if they are not otherwise described in the exclusion criteria AND there was agreement to allow by both the investigator and sponsor).
- Anti-tumor therapy (chemotherapy, antibody therapy, molecular targeted therapy, retinoid therapy, hormonal therapy [except for subjects with breast cancer], or investigational agent) within 28 days of study day 1; concurrent use of hormone deprivation therapy for hormone-refractory prostate cancer or breast cancer is permitted.
- Therapeutic or palliative radiation therapy within 2 weeks of study day 1. Subjects must have recovered from all radiotherapy related toxicity.
- Currently enrolled in another investigational device or drug study, or less than 28 days since ending another investigational device or drug study(s) or receiving other investigational agent(s).
- Other investigational procedures are excluded.
- Major surgery within 28 days of study day 1.
- Men and women of childbearing potential (WOCBP) who were unwilling to practice acceptable methods of birth control during treatment and for at least 7 days (women) or 7 days (men) after receiving the last dose of the compound of Formula I. Acceptable methods of highly effective birth control for women included sexual abstinence (refraining from heterosexual intercourse); vasectomy (women with a single male sexual partner) with testing showing there is no sperm in the semen; bilateral tubal ligation or occlusion; or intrauterine device. Acceptable methods of birth control for men included sexual abstinence (refraining from heterosexual intercourse); vasectomy with testing showing there is no sperm in the semen; bilateral tubal ligation or occlusion in the partner; or a condom (the female partner should also consider a form of birth control).
- Women who were lactating/breast feeding or who planned to breastfeed while on study through 7 days after receiving the last dose of study drug.
- Women with a positive pregnancy test.
- Women planning to become pregnant while on study through 7 days after receiving the last dose of study drug.
- Subject had known sensitivity to any of the products to be administered during dosing.
- Subject was not available for protocol-required study visits or procedures, to the best of the subject and investigator's knowledge.
- Subject had any kind of disorder that, in the opinion of the investigator, may have compromise the ability of the subject to give written informed consent and/or to comply with all required study procedures.
- History or evidence of any other clinically significant disorder, condition, or disease (with the exception of those outlined above) that, in the opinion of the investigator or physician would have posed a risk to subject safety or interfered with the study evaluation, procedures or completion.
- Use of known cytochrome P450 (CYP) 3A4 sensitive substrates (with a narrow therapeutic window), within 14 days or 5 half-lives of the drug or its major active metabolite, whichever is longer, prior to study day 1 that was not reviewed and approved by the principal investigator and the sponsor medical monitor.
- Use of strong inducers of CYP3A4 (including herbal supplements such as St. John's wort) within 14 days or 5 half-lives (whichever is longer) prior to study day 1 that was not reviewed and approved by the principal investigator and the sponsor medical monitor.
- History of other malignancy within the past 2 years, with the following exceptions:
   ∘ Malignancy treated with curative intent and with no known active disease present for ≥ 2 years before enrollment and felt to be at low risk for recurrence by the treating physician.
   ∘ Adequately treated non-melanoma skin cancer or lentigo maligna without evidence of disease.
   ∘ Adequately treated cervical carcinoma in situ without evidence of disease.
   ∘ Adequately treated breast ductal carcinoma in situ without evidence of disease.
   ∘ Prostatic intraepithelial neoplasia without evidence of prostate cancer.
   ∘ Adequately treated urothelial papillary non-invasive carcinoma or carcinoma in situ.
- Previous treatment with a direct KRAS^{G12C} inhibitor.

### Tumor Tissue Sample Collection for Central Laboratory Testing

Samples for biomarker analysis, e.g., to evaluate potential biomarkers that may correlate with treatment response, were collected. Subjects were not enrolled until results of the central laboratory testing had been reported and demonstrated that the subject has the *KRAS G12C* mutation.

For subjects with NSCLC an archival tumor sample (formalin fixed, paraffin embedded (FFPE) sample, collected within 5 years of enrollment) or pretreatment tumor biopsy (core needle or fine needle aspirates or excisional FFPE sample) was submitted prior to enrollment for central laboratory testing as well as subsequent exploratory biomarker analyses. Tumor tissue was allowed to be submitted to the central laboratory either as FFPE blocks or unstained slides.

*KRAS G12C* mutation testing was conducted using a therascreen^{®} KRAS RGQ PCR Kit (Qiagen). The kit is a real-time qualitative PCR assay performed on the Rotor-Gene Q MDx instrument for the detection of 7 somatic mutations in the human KRAS oncogene using DNA extracted from FFPE tissue. The mutations detected are: G12A, G12D, G12R, G12C, G12S, G12V, and G13D.

PD-L1 testing was conducted at the central labs using the Dako PharmDx 22C3 immunohistochemistry FDA-approved kit according to the instructions for use. More specifically, immunohistochemical evaluation of programmed death-ligand 1 (PD-L1) expression on tumor cells was performed using the PD-L1 IHC 22C3 pharmDx assay on the BOND III automated staining system (Leica Microsystems), according to the manufacturer's instructions for use (IFU). All slides were reviewed and scored by a board-certified pathologist trained to score the PD-L1 IHC 22C3 pharmDx assay. The reporting of tumor cell PD-L1 expression (% tumor proportion score) and the test result (positive/negative) was conducted in accordance with established companion diagnostic guidelines by the Food and Drug Administration (FDA) for 22C3.

Subject testing with the therascreena^{®}KRAS RGQ Assay and Dako PharmDx 22C3 took place at the NeoGenomics Central Testing Laboratory in Houston, Texas.

*STK11, KEAP1*, and *TP53* mutation testing was conducted using the Resolution Bioscience Resolution ctDx Lung^{™} assay that included 24 genes (including those actionable in NSCLC). Tissue samples were tested using Tempus xT 648 panel. Further, *STK11* and *KEAP1* mutation testing was also conducted using the Qiagen Comprehensive Cancer Panel (NGS). *STK11*/*KEAP1*/*TP53* co-mutation analysis determined mutational status from baseline tissue and/or plasma samples, and mutations include nonsense, missense, frameshift, or splice site mutations and insertion/deletions predicted to be loss-of-function, and excluded variants of unknown significance. Subjects with variant of uncertain significance (VUS) in *STK11, KEAP1*, or *TP53* were not included in the corresponding comparisons of mutant versus wild-type for each gene. A subject with any qualifying variant was considered mutated for that gene, and otherwise wild type. Due to platform differences in report content, the filtering approach was customized to each platform. Tempus' filtered molecular master file (MMF) was processed to retain non-synonymous variants (including splice site mutations, deletions, frame shifts), rearrangements, and copy number variations (CNVs). Excluded variants were those not in COSMIC or dbSNP with either non-pathogenic status and allele frequency at least 0.40 or one of the following cases: uncertain significance, benign, likely benign or germline. Germline status was provided by Tempus, based on matched normal samples. Resolution Biosciences' cfDNA NGS results were annotated using SnpEff v4.3t, and variants were further annotated with information from COSMIC (v92) and dbSNP (build 154) using SnpSift v4.3t. Variants were then filtered down to retain non-synonymous variants (including splice site mutations, deletions, frame shifts), ClinVar clinical significance of at least 4 (likely-pathogenic), indels, CNVs, and variants in at least 3 COSMIC samples. Excluded variants were those not in COSMIC or dbSNP with allele frequency at least 0.40 or ClinVar clinical significance of uncertain, benign or likely benign.

### Results

### Demographic and Baseline Characteristics

As shown in Table 1.1 below, a total of 126 subjects with *KRAS G12C*-mutated locally advanced or metastatic NSCLC were enrolled in the phase 2 portion of the CodeBreaK 100 Trail and had received ≥ 1 dose of the compound of Formula I as monotherapy (960 mg once daily). Of these, 123 subjects received ≥ 1 dose of the compound of Formula I and had ≥ 1 measurable lesion per RECIST 1.1 (based on central review) at baseline and were included in the full analysis set for efficacy assessments (Table 2.1). As of the data cutoff date of September 1, 2020, 69 of the 126 subjects (54.8%) with NSCLC were continuing participation in the study. Of the 57 subjects (45.2%) who discontinued the study, 47 subjects (37.3%) died and 10 subjects (7.9%) withdrew consent. 89 subjects (70.6%) had discontinued treatment with the compound of Formula I; the most common reasons for discontinuation were disease progression (70 subjects [55.6%]) and adverse event (11 subjects [8.7%]). Median duration (range) of treatment with the compound of Formula I was 24.1 (1.0, 52.1) weeks, with 47.6% and 28.6% of subjects receiving ≥ 6 and ≥ 9 months of treatment, respectively. The median relative dose intensity was 100%.

Of the 126 enrolled subjects with NSCLC, 81.7% were white and 50% were men. The median (range) age was 63.5 (37, 80) years. Most subjects had non-squamous NSCLC (99.2%) and stage IV disease at screening (96.0%). Per protocol eligibility criteria, subjects had a baseline Eastern Cooperative Oncology Group (ECOG) performance status of 0 or 1 (38 subjects [30.2%] and 88 subjects [69.8%], respectively). Subjects had received a median of 2 prior lines of anticancer therapy: 54 subjects (42.9%) received 1 prior line, 44 (34.9%) received 2 prior lines, 28 (22.2%) received 3 prior lines, and no subject received 4 or more. A total of 113 subjects (89.7%) received prior platinum-based chemotherapy and 115 subjects (91.3%) received prior anti-PD-1/PD-L1 immunotherapy therapy. A total of 102 subjects (81.0%) had received and progressed on treatment with both checkpoint inhibitors and platinum-based chemotherapy. Most subjects were former smokers (102 subjects, 81.0%) or current smokers (15 subjects, 11.9%). Overall, 7.1% had received and progressed on targeted small molecule therapies (none were approved therapies/combinations for actionable mutations). Based on available data reported by the study centers and consistent with the literature, a low number of subjects had co-mutations (10.3% of subjects for *TP53,* 5.6% for *STK11,* 2.4% for *EGFR,* and < 1.6% for all other co-mutations; no subjects had co-mutations in *ALK* or *ROS* - based on data available at local sites). Overall, 96.8% of subjects had metastatic disease. Per protocol eligibility criteria, while subjects with treated and stable brain metastases could enroll in the study, subjects with active brain metastases were excluded. This is in keeping with the standard of treating active brain metastases with surgery or radiation before initiating pharmacotherapy.

**Table 1.1.**

| **Baseline Characteristics** | | **Sotorasib 960mg, N=126** | | |
|---|---|---|---|---|
| Median age - years (range) | | 63.5 (37-80) | | |
| Smoking history - n (%)^{a} | | | | |
| | Never | 6 (4.8) | | |
| | Current or former | 117 (92.9) | | |
| ECOG performance status - n (%) | | | | |
| | 0 | 38 (30.2) | | |
| | 1 | 88 (69.8) | | |
| Prior lines of anticancer systemic therapy - n (%) | | | | |
| | 1-2 | 98 (77.8) | | |
| | 3 | 28 (22.2) | | |
| Type of prior anticancer systemic therapy - n (%) | | | | |
| | Platinum-based chemotherapy | 113 (89.7) | | |
| | PD-1 or PD-L1 inhibitors | 115 (91.3) | | |
| | Platinum-based chemotherapy and PD1/L1 inhibitors | 102 (81.0) | | |
| | Targeted therapies & other | 40 (31.7) | | |

Further, as of a March 15, 2021 data cutoff, a total of 126 patients with previously treated, KRAS p.G12C mutated NSCLC were enrolled and received at least 1 dose of sotorasib. According to independent central review, 2 patients did not have measurable lesions at baseline and were ineligible for response assessment. Among the remaining 124 patients, one patient did not have centrally confirmed KRAS p.G12C; this patient had stable disease and was included in the response assessments as prespecified by protocol. The data cutoff was March 15, 2021, with a median follow-up of 15.3 months (range, 1.1+ to 18.4, + indicates that the value includes patient data that were censored at data cutoff). The median duration of treatment was 5.5 months (range, 0.2 to 17.8). A total of 88 patients (69.8%) received sotorasib for 3 months or more, 60 (47.6%) for 6 months or more, and 41 (32.5%) for 9 months or more. Dose reduction occurred in 26 patients (20.6%). As of the cutoff, 103 patients (81.7%) had discontinued treatment with sotorasib; disease progression (n = 83, 65.9%) and adverse events regardless of attribution (n = 11, 8.7%) were the most common reasons for discontinuation.

Baseline characteristics are summarized in Table 1.2 and further detailed in Table 1.3. Among 126 patients enrolled, the median age was 63.5 years (range, 37 to 80), and 117 (92.9%) were current or former smokers. Patients had received a median of 2 prior lines of systemic anticancer therapy. Prior therapies included platinum-based chemotherapy (n = 113, 89.7%), checkpoint inhibitors (n = 116, 92.1%), and anti-angiogenic therapies (n = 25, 19.8%). A total of 102 patients (81.0%) received both platinum-based chemotherapy and PD-1 / L1 inhibitors.

**Table 1.2.**

| **Characteristics** | | | **All NSCLC patients, sotorasib 960mg (N = 126)** | |
|---|---|---|---|---|
| Median age (range) - years | | | 63.5 (37-80) | |
| Female sex - n (%) | | | 63 (50.0) | |
| Race - n (%) | | | | |
| | White | | 103 (81.7) | |
| | Asian | | 19 (15.1) | |
| | Black | | 2 (1.6) | |
| | Other | | 2 (1.6) | |
| Smoking history - n (%) | | | | |
| | Never | | 6 (4.8) | |
| | Current | | 15 (11.9) | |
| | Former | | 102 (81.0) | |
| | Missing | | 3 (2.4) | |
| ECOG performance status - n (%) | | | | |
| | 0 | | 38 (30.2) | |
| | 1 | | 88 (69.8) | |
| Brain metastasis - n (%) | | | | |
| | Yes | | 26 (20.6) | |
| | No | | 100 (79.4) | |
| Histology subtype - n (%) | | | | |
| | Squamous cell carcinoma | | 1 (0.8) | |
| | Adenocarcinoma | | 120 (95.2) | |
| | Large cell carcinoma | | 3 (2.4) | |
| | Bronchoalveolar carcinoma | | 2 (1.6) | |
| Metastatic disease - n (%) | | | | |
| | Yes | | 122 (96.8) | |
| | No | | 4 (3.2) | |
| Prior line of anticancer systemic therapy - n (%) | | | | |
| | 1 | | 54 (42.9) | |
| | 2 | | 44 (34.9) | |
| | 3 | | 28 (22.2) | |
| Type of prior anticancer systemic therapy - n (%) | | | 115 (91.3) | |
| | Chemotherapy^{a} | | 113 (89.7) | |
| | | *Platinum-based chemotherapy* | 116 (92.1) | |
| | Checkpoint inhibitors | | 115 (91.3) | |
| | | *Anti PD-1 or anti PD-L1* | 102 (81.0) | |
| | Platinum-based chemotherapy and PD1/L1 inhibitors | 25 (19.8) | | |
| | Anti-angiogenic monoclonal antibodies | 9 (7.1) | | |
| | Targeted small molecules^{b} | 1 (0.8) | | |
| | Other^{c} | | | |

**Table 1.3.**

| | | Phase 2 NSCLC 960 mg QD Fasted (N = 126) | | |
|---|---|---|---|---|
| Weight (kg) | | | | |
| | n | 126 | | |
| | Mean | 71.08 | | |
| | SD | 17.14 | | |
| | Median | 70.65 | | |
| | Q1, Q3 | 57.70, 83.00 | | |
| | Min, Max | 36.8, 122.7 | | |
| Height (cm) | | | | |
| | n | 123 | | |
| | Mean | 167.83 | | |
| | SD | 9.20 | | |
| | Median | 168.80 | | |
| | Q1, Q3 | 161.00, 175.00 | | |
| | Min, Max | 146.0, 188.0 | | |
| Type of cancer - n (%) | | | | |
| | Non-small cell lung | 126 (100.0) | | |
| Disease stage at initial diagnosis - n (%) | | | | |
| | Stage I | 11 (8.7) | | |
| | Stage II | 14 (11.1) | | |
| | Stage III | 22 (17.5) | | |
| | Stage IV | 78 (61.9) | | |
| | Missing | 1 (0.8) | | |
| Disease stage at screening - n (%) | | | | |
| | Stage I | 0 (0.0) | | |
| | Stage II | 0 (0.0) | | |
| | Stage III | 5 (4.0) | | |
| | Stage IV | 121 (96.0) | | |
| Differentiation - n (%) | | | | |

| | | | | Phase 2 NSCLC 960 mg QD Fasted (N = 126) |
|---|---|---|---|---|
| | Well differentiated | | | 6 (4.8) |
| | Moderately differentiated | | | 15 (11.9) |
| | Poorly differentiated | | | 24 (19.0) |
| | Undifferentiated | | | 0 (0.0) |
| | Other | | | 0 (0.0) |
| | Unknown | | | 81 (64.3) |
| Histopathology type - n (%) | | | | |
| | Squamous | | | 1 (0.8) |
| | | Adenosquamous carcinoma | | 0 (0.0) |
| | | Squamous cell carcinoma | | 1 (0.8) |
| | Non-squamous | | | 125 (99.2) |
| | | Adenocarcinoma | | 120 (95.2) |
| | | | Mucinous | 8 (6.3) |
| | | Large cell carcinoma | | 3 (2.4) |
| | | Bronchoalveolar carcinoma | | 2 (1.6) |
| | | Sarcomatoid | | 0 (0.0) |
| | | Undifferentiated | | 0 (0.0) |
| | Other | | | 0 (0.0) |
| Number of body sites of metastatic disease - n (%) | | | | |
| | 0 | | | 4 (3.2) |
| | 1 | | | 51 (40.5) |
| | 2 | | | 30 (23.8) |
| | 3 | | | 24 (19.0) |
| | >3 | | | 17 (13.5) |
| Liver metastasis - n (%) | | | | |
| | Yes | | | 26 (20.6) |
| | No | | | 100 (79.4) |
| Brain metastasis - n (%) | | | | |
| | Yes | | | 26 (20.6) |
| | No | | | 100 (79.4) |
| Bone metastasis - n (%) | | | | |
| | Yes | | | 61 (48.4) |
| | No | | | 65 (51.6) |

| | | Phase 2 NSCLC 960 mg QD Fasted (N = 126) | | |
|---|---|---|---|---|
| Region - n (%) | | | | |
| | North America | 79 (62.7) | | |
| | Europe | 30 (23.8) | | |
| | Asia | 12 (9.5) | | |
| | Rest of the world | 5 (4.0) | | |

### Overall Efficacy Results

The key efficacy results for tumor response in subjects with NSCLC from the phase 2 portion of the CodeBreaK 100 Trial (full analysis set) are provided in Table 2.1 below.

Based on blinded independent central review using RECIST 1.1, the ORR among subjects with NSCLC in the phase 2 portion of the CodeBreaK 100 Trial was 37.4% (95% CI: 28.8, 46.6) with 46 of the 123 evaluable subjects achieving a complete or a partial response, including 2 subjects (1.6%) with a complete response and 44 subjects (35.8%) with a partial response. The lower limit of the 95% CI excluded the prespecified benchmark ORR of 23%. Among the 46 responders in the phase 2 NSCLC group, the median time to response was 1.35 months (range: 1.2 to 6.1). Median duration of response (DOR) was 8.4 months (95% CI: 6.9, 8.4). As of the data cutoff date, among the 46 responders, 24 subjects (52.2%) were still receiving treatment with ongoing response; the median follow-up time for DOR was 6.9 months (range: 1.3 to 8.4+).

Among the 99 subjects who had prior treatment with both platinum-based chemotherapy and anti-PD-1 or anti-PD-L1, ORR was 32.3% (95% CI: 23.3, 42.5) and median duration of response was not estimable (95% CI: 6.9 months, not estimable). This subset of subjects had received the most effective available therapies in NSCLC, hence satisfactory treatment options are limited.

Thus, the observed ORR, which is rapid and durable, represents a clinically meaningful benefit when considering the intended patient population with life-threatening disease and limited available therapies.

The PFS and OS results provide additional evidence of the efficacy of the compound of Formula I. The median follow-up time for PFS was 8.3 months (range: 0.3 to 11.5+). Median PFS based on central review was 6.7 months (95% CI: 4.9, 8.1); the Kaplan-Meier estimate for PFS at 6 months was 51.5% and 36.2% at 9 months. Median follow-up time for OS was 9.3 months (range: 1.1 to 12.2). Median OS was 12.0 months (95% CI: 9.5, not estimable); the Kaplan-Meier estimate for OS at 6 months was 75.5% and 63.4% at 9 months. As of the data cutoff date (September 1, 2020), 69 of the 126 subjects (54.8%) were alive at last follow-up visit, 9 (7.1%) had withdrawn consent, and 48 (38.1%) had died.

Furthermore, the compound of Formula I was well tolerated with no deaths attributed to treatment and low incidence of grade 3 or 4 treatment-related adverse events, treatment discontinuation, and dose modification.

**Table 2.1.**

| Summary of Objective Response Based on Blinded Independent Central Review | | | | | Phase 2 NSCLC 960 mg QD Fasted (N = 123) |
|---|---|---|---|---|---|
| Best overall response - n (%) | | | | | |
| | Complete response (CR) | | | | 2 (1.6) |
| | Partial response (PR) | | | | 44 (35.8) |
| | Stable disease (SD) | | | | 53 (43.1) |
| | Progressive disease (PD) | | | | 20 (16.3) |
| | Not evaluable (NE) | | | | 2 (1.6) |
| | Not done | | | | 2 (1.6) |
| Objective response rate (ORR) | | | | | |
| | Number of overall responders - N1 (%) | | | | 46 (37.4) |
| | 95% CI^{a} | | | | (28.84, 46.58) |
| Disease control rate (DCR) - n (%) | | | | | 99 (80.5) |
| | 95% CI^{a} | | | | (72.37, 87.08) |
| Duration of objective response (DOR)^{b} | | | | | |
| | Observed duration ≥ 3 months - n (%) | | | | 35 (76.1) |
| | Observed duration ≥ 6 months - n (%) | | | | 23 (50.0) |
| | Subject status - n (%) | | | | |
| | | Events | | | 14 (30.4) |
| | | | Progressive disease | | 13 (28.3) |
| | | | Death | | 1 (2.2) |
| | | | | Related to COVID-19 | 0 (0.0) |
| | | Censored | | | 32 (69.6) |
| | | | On study without disease progression | | 28 (60.9) |
| | | | No evaluable post-baseline disease assessment | | 0 (0.0) |
| | | | Missed more than 1 consecutive assessments | | 0 (0.0) |
| | | | | Related to COVID-19 | 0 (0.0) |
| | | | Started new anti-cancer therapy | | 3 (6.5) |
| | | | Withdrew consent | | 1 (2.2) |
| | | | | Related to COVID-19 | 0 (0.0) |
| | | | Off study due to sponsor decision | | 0 (0.0) |
| | | | | Related to COVID-19 | 0 (0.0) |
| | | | Lost to follow-up | | 0 (0.0) |
| | | | | Related to COVID-19 | 0 (0.0) |

**Table 2.1. Continued.**

| Summary of Objective Response Based on Blinded Independent Central Review | | | | Phase 2 NSCLC 960 mg QD Fasted (N = 123) |
|---|---|---|---|---|
| | Duration of response (KM) (months) | | | |
| | | 25th percentile (95% CI) | | 6.8 (3.5, 7.1) |
| | | Median (95% CI) | | 8.4 (6.9, 8.4) |
| | | 75th percentile (95% CI) | | 8.4 (NE, NE) |
| | | Min, Max (+ for censored) | | 1.3+, 8.4 |
| | | Kaplan-Meier estimate (95% CI)^{c} | | |
| | | | At 3 months | 89.9 (75.3, 96.1) |
| | | | At 6 months | 76.2 (59.1, 86.9) |
| | | | At 9 months | 0.0 (NE, NE) |
| | | | At 12 months | 0.0 (NE, NE) |
| | Follow-up time for DOR^{d} (KM) (months) | | | |
| | | 25th percentile (95% CI) | | 5.5 (2.8, 6.7) |
| | | Median (95% CI) | | 6.9 (5.6, 7.0) |
| | | 75th percentile (95% CI) | | 7.1 (7.0, 8.1) |
| | | Min, Max (+ for censored) | | 1.3, 8.4+ |
| Time to objective response (months)^{b} | | | | |
| | Number of subjects with objective response | | | 46 |
| | Mean (SD) | | | 1.95 (1.23) |
| | Median | | | 1.35 |
| | Q1, Q3 | | | 1.25, 2.69 |
| | Min, Max | | | 1.2, 6.1 |

| | | | | |
|---|---|---|---|---|
| Phase 2 data cut-off date September 1, 2020. As of the data cutoff date, a total of 69 of 126 subjects (54.8%) were continuing participation in the study. CI = confidence interval; KM = Kaplan-Meier; N = number of subjects in the analysis set; n = number of subjects with observed data; NE = not estimable; NSCLC = non-small cell lung cancer; QD = once daily; RECIST 1.1 = response evaluation criteria in solid tumors; Months are derived as days x (12/365.25). Best Overall Response for a subject is the best observed disease response per RECIST1.1 based on central review. ORR is defined as the proportion of subjects with complete response or partial response and confirmation after at least 4 weeks. DCR is defined as the proportion of subjects with complete response or partial response with confirmation or stable disease ≥ 5 weeks. DOR is defined as time from first evidence of complete response or partial response to disease progression or death due to any cause, among responders. Time to response is defined as time from the first dose of sotorasib until the first evidence of complete response or partial response, among responders. ^{a} Exact 95% CI was calculated using the Clopper Pearson method ^{b} Time to response and duration of response are calculated among confirmed responders N1. ^{c} 95% CIs are based on estimated variance for log-log transformation of the KM survival estimate. ^{d} Follow-up time is measured by reversing the status indicator for censored and events. Events marked "Related to COVID-19" were identified from available information collected on the case report form (CRF) and protocol deviation data. | | | | |

As of the March 15, 2021 data cutoff, the following efficacy information is available:
Of the 124 patients evaluated for response, 4 (3.2%) had a complete response, and 42 (33.9%) had a partial response, resulting in an objective response rate of 37.1% (95% Cl, 28.6 to 46.2). The objective response rate was independent of KRAS p.G12C mutational allele frequency (MAF), with an odds ratio of 1.11 (95% CI: 0.88, 1.39) for each 0.10 increase in MAF. Disease control (complete response, partial response, or stable disease) was achieved in 100 patients (80.6%, 95% Cl, 72.6 to 87.2) (Table 2.2). Tumor shrinkage of any magnitude was observed in 102 patients (82%); median percentage of best tumor shrinkage among all responders was 60%. Disease progression was the best overall response in 20 patients. A total of 4 patients were not evaluable (n = 2) or had missing scans (n = 2).

**Table 2.2.**

| **Response** | | | **Independent Central Review (N = 124)** |
|---|---|---|---|
| Objective response rate - % (95% Cl) | | | 37.1 (28.60, 46.23) |
| Disease control rate - % (95% Cl) | | | 80.6 (72.58, 87.19) |
| Best response - n (%) | | | |
| | Complete response | | 4 (3.2) |
| | Partial response | | 42 (33.9) |
| | Stable disease | | 54 (43.5) |
| | Progressive disease | | 20 (16.1) |
| | Not evaluable | | 2 (1.6) |
| | Missing scan | | 2 (1.6) |
| Duration of response | | | |
| | Patients with a response - n (%) | | |
| | Median duration of response - months (95% Cl) | | 46 (37.1) |
| | | | 11.1 (6.9, NE) |
| | Kaplan-Meier estimate (95% Cl) | | |
| | | 3 months | 90.5 (76.7, 96.3) |
| | | 6 months | 70.8 (54.3, 82.2) |
| | | 9 months | 57.3 (40.4, 71.0) |

Objective response rate was consistent across patient subgroups prespecified by the number of prior lines of therapy and prior treatment with anti-PD-1 or anti-PD-L1 therapy (Table 2.3.).

**Table 2.3.**

| | **ORR (95% CI)** |
|---|---|
| **Prior Lines of Therapy** | |
| 1 (53) | 39.6 (26.5, 54.0) |
| ≥ 2 (71) | 35.2 (24.2, 47.5) |
| **Prior Anti-PD-1/PD-L1 Therapy** | |
| Yes (113) | 36.3 (27.4, 45.9) |
| No (11) | 45.5 (16.7, 76.6) |

Among 46 patients who had an objective response, the median time to response was 1.4 months, and the median duration of response was 11.1 months (95% Cl, 6.9 to not evaluable). Response was observed at the first tumor assessment around week-6 in 71.7% of the responders (33/46). As of the cutoff, 16 (34.7%) responders remained on treatment without progression. The 3-, 6-, and 9-month Kaplan-Meier estimates for duration of response among those who responded were 90.5 % (95% Cl, 76.7 to 96.3), 70.8% (95% Cl, 54.3 to 82.2), and 57.3 % (95% Cl, 40.4 to 71.0), respectively.

The median progression-free survival among 124 evaluable patients was 6.8 months (95% Cl, 5.1 to 8.2). The 6-month and 9-month Kaplan-Meier estimates for PFS were 52.2% (95% Cl, 42.6 to 60.9) and 37.5% (95% Cl, 28.4 to 46.5). The median overall survival among all 126 patients enrolled was 12.5 months (95% CI, 10.0 to not evaluable).

According to local investigator assessment, which included all 126 patients, 2 (1.6%) had a complete response, 37 (29.4%) had a partial response, 69 (54.8%) had stable disease, and 15 (11.9%) had disease progression (Table 2.4).

**Table 2.4.**

| **Response** | | | **Investigator Assessment (N = 126)** |
|---|---|---|---|
| Objective response rate - % (95% Cl) | | | 31.0 (23.0, 39.8) |
| Disease control rate - % (95% Cl) | | | 85.7 (78.4, 91.3) |
| Best response - n (%) | | | |
| | Complete response | | 2 (1.6) |
| | Partial response | | 37 (29.4) |
| | Stable disease | | 69 (54.8) |
| | Progressive disease | | 15 (11.9) |
| | Not evaluable | | 2 (1.6) |
| | Missing scan | | 1 (0.8) |
| Duration of response | | | |
| | Patients with a response - n (%) | | 39 (31.0) |
| | Median duration of response - months (95% Cl) | | 11.1 (8.4, 12.6) |
| | Kaplan-Meier estimate (95% Cl) | | |
| | | 3 months | 94.7 (80.6, 98.7) |
| | | 6 months | 78.6 (61.7, 88.7) |
| | | 9 months | 62.0 (44.4, 75.5) |

### Subgroup Efficacy Results

Analyses were conducted to explore the treatment effect across subgroups (e.g., age, race, prior lines of anticancer therapy, prior immunotherapy treatment, ECOG status, histopathology type, disease status, presence of liver or brain metastasis, smoking history, region, best response on last prior therapy) among subjects with NSCLC in phase 2 (Table 3). While subgroup analysis interpretation may be limited, because of the small sample size of each subgroup, certain notable effects and trends were observed:

### Prior Platinum-Based Chemotherapy

The ORR was notably higher for subjects who had not received prior platinum-based chemotherapy compared with those who had received it and with the overall subject population (69.2% [9 of 13 subjects] vs 33.6% [37 of 110 subjects] and 37.4% [46 of 123 subjects], respectively).

### Brain Metastasis

The ORR was notably higher for subjects without brain metastasis than those with brain metastasis (43.3% [42 of 97 subjects] versus 15.4% [4 of 26 subjects]). The ORR was notably lower for subjects with brain metastasis compared with overall subject population (15.4% [4 of 26 subjects] versus 37.4% [46 of 123 subjects]).

### Bone Metastasis

A trend to higher ORR was observed for subjects without bone metastasis compared to those with bone metastasis (41.5% [27 of 65 subjects] versus 32.8% [19 of 58 subjects]).

### PD-L1 Protein Expression

Table 3 provides PD-L1 protein expression results obtained locally. A trend to higher ORR was observed in subjects with lower PD-L1 protein expression as measured by the tumor proportion score (TPS). The group with a PD-L1 TPS ≥50% has a lower ORR compared to the overall response (26.5% [9 of 34 subjects] versus 37.4% [46 of 123 subjects]), whereas the group with a PD-L1 TPS of <1% and the group with a PD-L1 TPS o f≥1% and <50% both have a higher ORR compared to the overall response (48.5% [16 of 33 subjects] and 40.9% [9 of 22 subjects] versus 37.4% [46 of 123 subjects]).

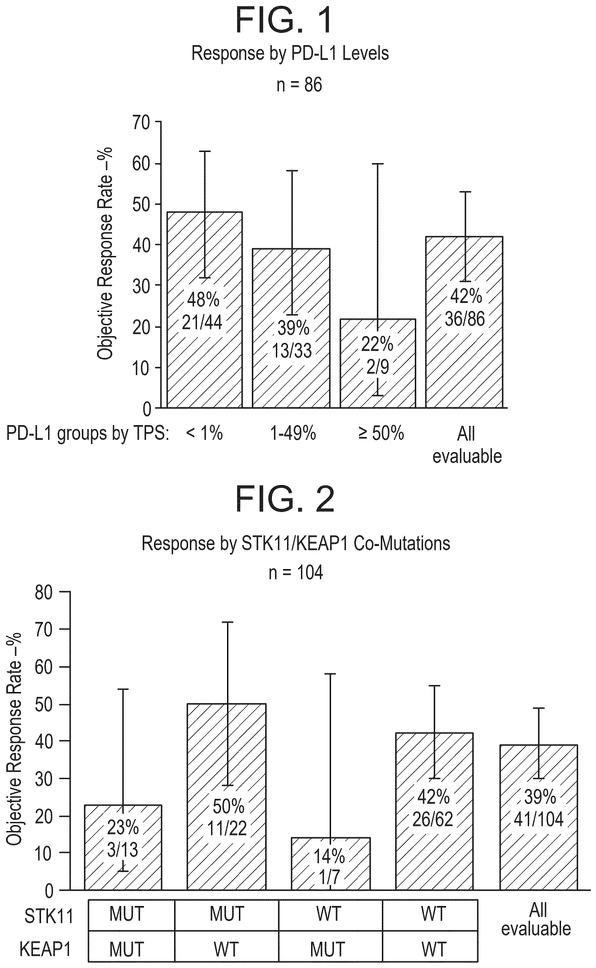

Retrospective PD-L1 central laboratory testing was conducted using the Dako PharmDx 22C3 immunohistochemistry FDA-approved kit according to the instructions for use on a subject population based on a data cutoff on December 1, 2020, which assessed 124 subjects evaluable for efficacy by central review, who had available biomarker data. This retrospective analysis shown in Figure 1 confirms the trend observed with the locally obtained results discussed above (see Table 3). Specifically, the group with a PD-L1 TPS ≥50% has a lower ORR compared to the overall response (22% [2 of 9 subjects] versus 42% [36 of 86 subjects]), whereas the group with a PD-L1 TPS of <1% and the group a PD-L1 TPS of ≥1% and <50% both have a higher or similar ORR compared to the overall response (48% [21 of 44 subjects] and 39% [13 of 33 subjects] versus 42% [36 of 86 subjects]).

### STK11/KEAP1/TP53 Co-Occurring Mutations

The ORR (%, non-responders/responders) by *STK11*/*KEAP1* co-occurring mutations in a subject population (N=124) evaluable for efficacy per central review, who had available biomarker data (N=104) (data cutoff December 1, 2020) is shown in Figure 2. *STK11*/*KEAP1* co-mutation analysis determined mutational status from baseline tissue and/or plasma samples, and mutations include nonsense, missense, frameshift, or splice site mutations and insertion/deletions predicted to be loss-of-function, and excluded variants of unknown significance. For clarification, the term "co-occurring mutations" refers to either or both of *STK11 and KEAP1* have mutations that co-occur with the *KRAS G12C* mutation.

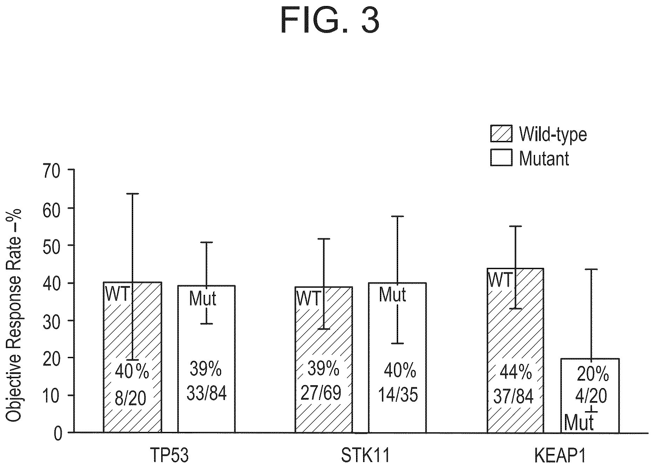

As discussed hereinabove, *STK11* and *KEAP1* co-occurring mutations have been associated with lower overall survival (OS) in *KRAS* mutant NSCLC (see Background). While OS remains the gold standard in clinical studies, analyses to explore the association between ORR and survival have demonstrated patient-level and study-level associations between ORR, PFS, and OS (Blumenthal et al, 2015; Clarke et al, 2015). Against this backdrop it is notable that clinically meaningful ORRs were observed across *STK11*/*KEAP1* co-occurring mutation profiles. In particular, the *KEAP1* co-occurring loss-of-function mutation, independent from the *STK11* mutational status (loss-of-function or wild-type), demonstrated ORR of 20% (4 of 20 subjects). Further, it is especially notable that the *STK11* co-occurring loss-of-function mutation, independent from the KEAP1 mutational status (loss-of-function or wild type), demonstrated a similar ORR when compared to the overall response in this subject population (40% [14 of 35 subjects] versus 39% [41 of 104 subjects], see also Figure 3). It is even more notable that the subject group with a *STK11* co-occurring loss-of-function mutation and a *KEAP1* wild-type or *STK11* wildtype and *KEAP1* wildtype shows a higher ORR when compared, respectively, to the overall response in this subject population (50% [11 of 22 subjects] versus 39% [41 of 104 subjects]) and (42% [26 of 62 subjects] vs 39% [41 of 104 subjects]).

Figure 3, which has been generated based on the March 15, 2021 data cutoff, shows the response by co-occurring mutation for *TP53, STK11,* and *KEAP1* (wild type v. mutant). The ORR for *TP53* and *STK11* (as discussed above) showed no notable difference between wild type and mutants, while a higher ORR was observed for *KEAP1* wild type compared to subjects with a *KEAP1* mutant. See also Table 4. As shown in Table 5, longer survival with sotorasib was seen in *STK11*-mutant group with concurrent wild-type *KEAP1,* whereas *KEAP1* -mutant groups appeared to derive less benefit, with the limitation of small sample size and the exploratory nature of this study.

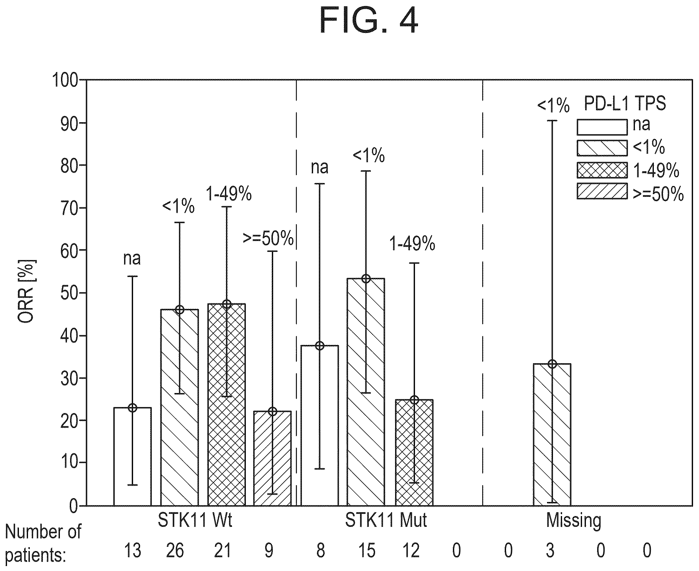

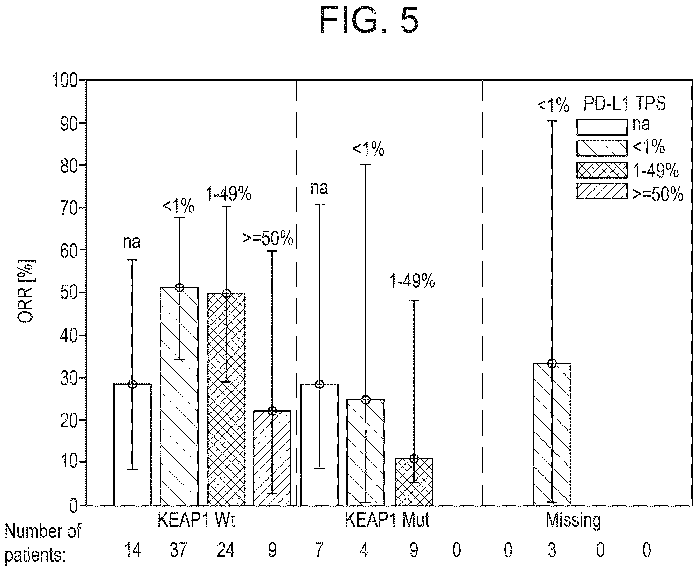

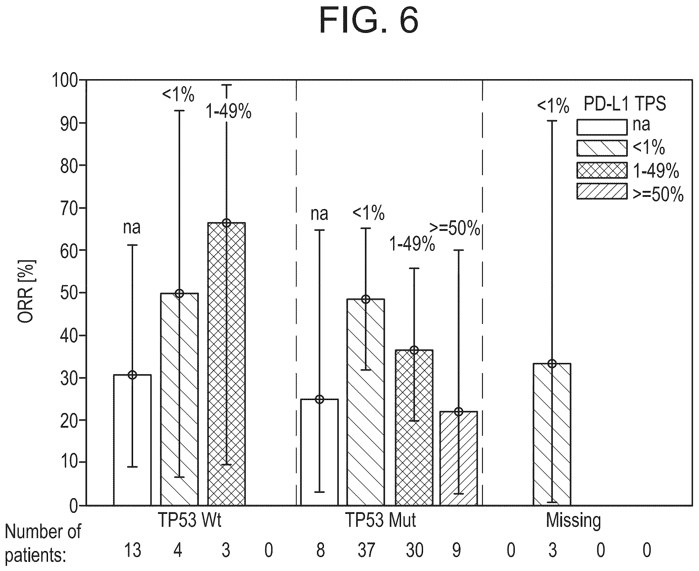

Figures 4, 5, and 6 show the objective response rate by PD-L1 expression level groups by TPS overlaid by *STK11, KEAP1,* and *TP53* co-occurring mutations (data cutoff March 15, 2021).

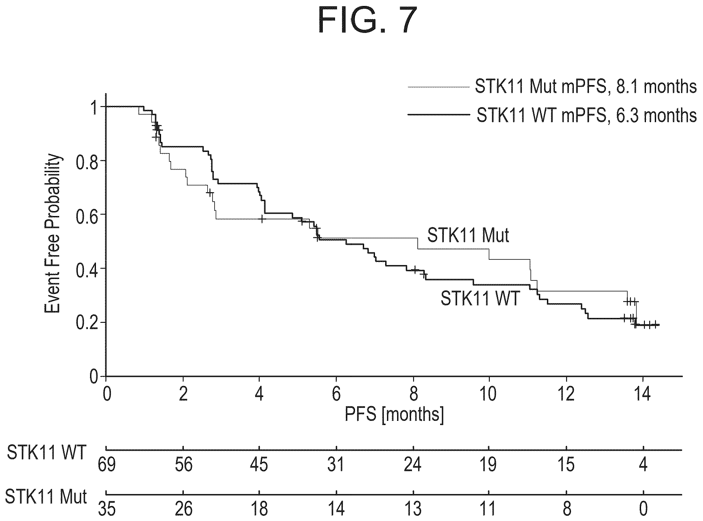

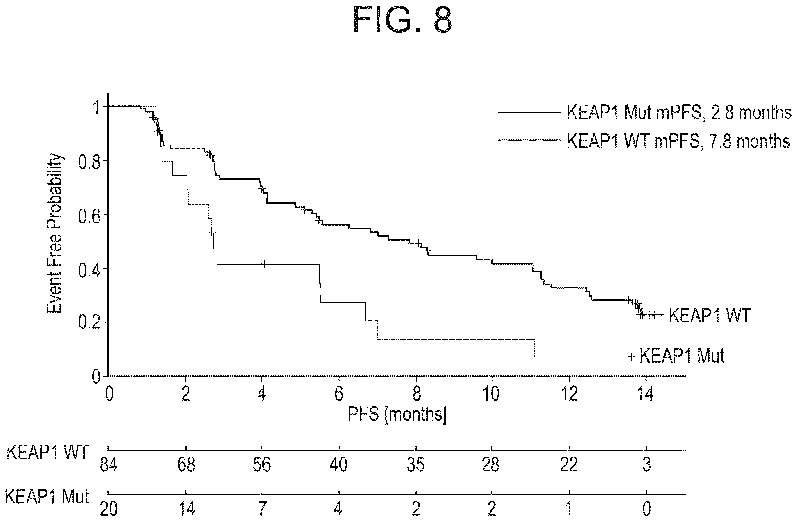

Further, Figures 7 and 8 show the progression free survival (PFS) by *STK11* and *KEAP1* mutational status (data cutoff March 15, 2021).

### Tumor Mutational Burden

As of the March 15, 2021 cutoff, in the patient subset assessed for tumor mutational burden (n = 84), response rate was 42% (95% CI, 30% to 55%) for the tumor mutational burden low group (<10 Mut/Mb) and 40% (95% CI, 16% to 68%) for the tumor mutational burden high group (≥10 Mut/Mb) (see Table 6).

**Table 3. Objective Response Rate Based on Central Review by Subgroup**

| Subgroup | | No. of subjects | | ORR (95% Cl) (%) |
|---|---|---|---|---|
| Overall | | 123 | 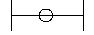 | 37.4 (28.8, 46.6) |
| Age at baseline | | | | |
| | < 65 years | 65 | 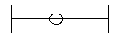 | 32.3 (21.2, 45.1) |
| | >= 65 years | 58 | 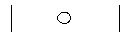 | 43.1 (30.2, 56.8) |
| Prior lines of anti-cancer therapy | | | | |
| | 1 | 53 | 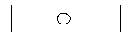 | 41.5 (28.1, 55.9) |
| | 2 | 43 | 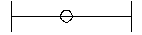 | 32.6 (19.1, 48.5) |
| | >2 | 27 | 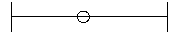 | 37 (19.4, 57.6) |
| Prior anti PD-1 or anti PD-L1 | | | | |
| | Yes | 112 | 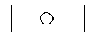 | 36.6 (27.7, 46.2) |
| | No | 11 | 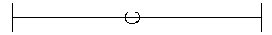 | 45.5 (16.7, 76.6) |
| Prior platinum-base chemotherapy | | | | |
| | Yes | 110 | 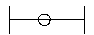 | 33.6 (24.9, 43.3) |
| | No | 13 | 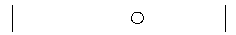 | 69.2 (38.6, 90.9) |
| Prior platinum-base chemotherapy and prior anti PD-1 or anti PD-L1 | | | | |
| | Yes | 99 | 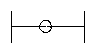 | 32.3 (23.3, 42.5) |
| | No | 24 | 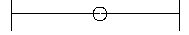 | 58.3 (36.6, 77.9) |
| PD-L1 protein expression | | | | |
| | < 1% | 33 | 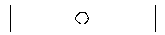 | 48.5 (30.8, 66.5) |
| | >= 1% and < 50% | 22 | 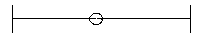 | 40.9 (20.7, 63.6) |
| | >= 50% | 34 | 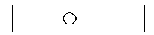 | 26.5 (12.9, 44.4) |
| ECOG status at baseline | | | | |
| | 0 | 37 | 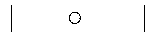 | 43.2 (27.1, 60.5) |
| | 1 | 86 | 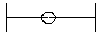 | 34.9 (24.9, 45.9) |
| Race | | | | |
| | White | 101 | 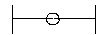 | 41.6 (31.9, 51.8) |
| | Black | 2 | 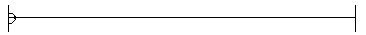 | 0 (0, 84.2) |
| | Asian | 18 | 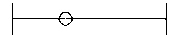 | 16.7 (3.6, 41.4) |
| | Other | 2 | 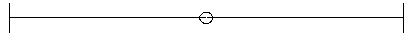 | 50 (1.3, 98.7) |
| | | | 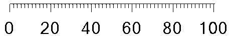 | |
| | | | Response rate | |

**Table 3. Continued**

| Subgroup | | No. of subjects | | ORR (95% CI) (%) |
|---|---|---|---|---|
| Sex | | | | |
| | Male | 61 | 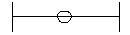 | 42.6 (30, 55.9) |
| | Female | 62 | 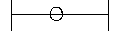 | 32.3 (20.9, 45.3) |
| Histopathology type | | | | |
| | Squamous | 1 | 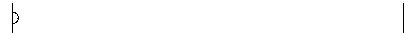 | 0 (0, 97.5) |
| | Non-squamous | 122 | 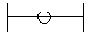 | 37.7 (29.1, 46.9) |
| Metastatic | | | | |
| | Yes | 119 | 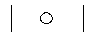 | 37 (28.3, 46.3) |
| | No | 4 | 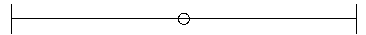 | 50 (6.8, 93.2) |
| Liver metastasis | | | | |
| | Yes | 26 | 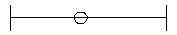 | 34.6 (17.2, 55.7) |
| | No | 97 | 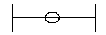 | 38.1 (28.5, 48.6) |
| Brain metastasis | | | | |
| | Yes | 26 | 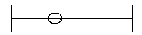 | 15.4 (4.4, 34.9) |
| | No | 97 | 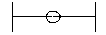 | 43.3 (33.3, 53.7) |
| Bone metastasis | | | | |
| | Yes | 58 | 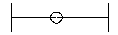 | 32.8 (21, 46.3) |
| | No | 65 | 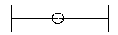 | 41.5 (29.4, 54.4) |
| Smoking history | | | | |
| | Never | 5 | 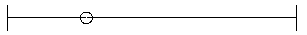 | 20 (0.5, 71.6) |
| | Current | 15 | 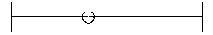 | 26.7 (7.8, 55.1) |
| | Former | 100 | 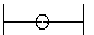 | 41 (31.3, 51.3) |
| Region | | | | |
| | North America | 79 | 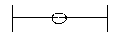 | 44.3 (33.1, 55.9) |
| | Europe | 28 | 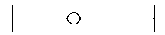 | 28.6 (13.2, 48.7) |
| | Asia | 11 | 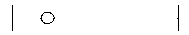 | 9.1 (0.2, 41.3) |
| | Rest of the world | 5 | 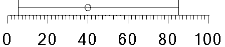 | 40 (5.3, 85.3) |
| | | | Response rate | |

| | | | | |
|---|---|---|---|---|
| ECOG = Eastern Cooperative Oncology Group; NSCLC = non-small cell lung cancer; PD-1 = programmed cell death-1; PD-L1 = programmed death-ligand 1. | | | | |

**Table 4. Analyses of PD-L1 and Co-Occurring Mutations in STK11, KEAP1, and TP53**

| | | | | | **95%CI** | |
|---|---|---|---|---|---|---|
| | **PD-L1 TPS** | **N** | **OR events** | **ORR** | **low** | **high** |
| | < 1% | 44 | 21 | 48% | 32% | 63% |
| | 1-49% | 33 | 13 | 39% | 23% | 58% |
| | ≥ 50% | 9 | 2 | 22% | 3% | 60% |
| | all evaluable | 86 | 36 | 42% | 31% | 53% |

| | | | | | **95%CI** | |
|---|---|---|---|---|---|---|
| **Gene** | **Mutation status** | **N** | **OR events** | **ORR** | **low** | **high** |
| *TP53* | wt | 20 | 8 | 40% | 19% | 64% |
| *TP53* | mut | 84 | 33 | 39% | 29% | 51% |
| *STK11* | wt | 69 | 27 | 39% | 28% | 52% |
| *STK11* | mut | 35 | 14 | 40% | 24% | 58% |
| *KEAP1* | wt | 84 | 37 | 44% | 33% | 55% |
| *KEAP1* | mut | 20 | 4 | 20% | 6% | 44% |

| | | | | | **95%CI** | |
|---|---|---|---|---|---|---|
| ***KEAP1*** | ***STK11*** | **N** | **OR events** | **ORR** | **low** | **high** |
| mut | mut | 13 | 3 | 23% | 5% | 54% |
| wt | mut | 22 | 11 | 50% | 28% | 72% |
| mut | wt | 7 | 1 | 14% | 0% | 58% |
| wt | wt | 62 | 26 | 42% | 30% | 55% |
| all evaluable | | 104 | 41 | 39% | 30% | 49% |

| | | | | | | |
|---|---|---|---|---|---|---|
| wt = wild type, mut = mutant | | | | | | |

**Table 5. mPFS and mOS by Co-occurring Mutations in both STK11 and KEAP1**

| ***KEAP1*** | ***STK11*** | N | mPFS, month (95% CI) | mOS, month (95% CI) |
|---|---|---|---|---|
| mut | mut | 13 | 2.6 (1.4, 11.1) | 4.8 (2.1, 10.8) |
| wt | mut | 22 | 11.0 (2.8, NE) | 15.3 (4.8, NE) |
| mut | wt | 7 | 5.5 (0, 7.0) | 7.5 (0, NE) |
| wt | wt | 62 | 6.8 (4.0, 11.0) | NE (NE, NE) |
| all evaluable | | 104 | 6.3 (4.1, 8.3) | 6.3 (4.1, 8.3) |

| | | | | |
|---|---|---|---|---|
| wt = wild type, mut = mutant | | | | |

**Table 6. Analysis of Tumor Mutational Burden (TMB) and Objective Response to Sotorasib**

| | **ORR (95% CI)** |
|---|---|
| **TMB Level** | |
| Low, <10 Mut/Mb (69) | 42.0 (30.2, 54.5) |
| High, ≥10 Mut/Mb (15) | 40.0 (16.3, 67.7) |

### EXAMPLE 2: A Phase 2, Multicenter, Open-label Study of Sotorasib (AMG 510) in Subjects with Stage IV NSCLC Whose Tumors Harbor a KRASG12C Mutation in Need of First-Line Treatment (CodeBreaK 201)

### Overall Design

An open-label, multicenter phase 2 study to explore the anti-tumor effect of sotorasib monotherapy in subjects with metastatic non-small cell lung cancer (NSCLC) with KRAS p.G12C mutation whose tumors express < 1% programmed death-ligand 1 (PD-L1) and/or have a serine/threonine kinase 11 (STK11) mutation in need of first line treatment is set up. Subjects are randomized in a 1:1 design for treatment with sotorasib at 960 mg orally (PO) daily (QD), stratified by known presence of STK11 mutation. The study is listed under ClinicalTrials.gov Identifier: NCT04933695 (see https://clinicaltrials.gov/ct2/show/NCT04933695; version of October 13, 2021; last accessed December 15, 2021).

An independent Data Review Team (DRT) will monitor the study. A non-binding futility guideline based on Bayesian posterior probability will be used to facilitate termination in the event of inadequate efficacy. Subjects are treated until disease progression as confirmed by Blinded Independent Central Review (BICR), unacceptable toxicity, withdrawal of informed consent, or death, whichever occurs first. All subjects should have a safety follow-up (SFU) visit approximately 30 (+ 7) days after the end of the last dosing interval of protocol-required therapies or before any new antitumor treatment is started. Following the SFU visit, subjects will be followed in the long-term follow-up (LTFU) phase of the study in clinic or via telephone every 12 weeks (± 2 weeks) for assessment of survival and documentation of anti-cancer treatment until death, withdrawal of consent, or end of study, whichever occurs first. Subjects who discontinue sotorasib for reasons other than radiographic disease progression will have LTFU imaging for disease status until disease progression is documented radiographically per Response Evaluation Criteria in Solid Tumors (RECIST) 1.1, initiate a non study cancer treatment, withdrawal of consent, or end of study, whichever occurs first.

Additional plasma/blood/tissue biopsy samples are collected for exploratory biomarkers for identification and quantification of biomarker expression at protein, RNA, and DNA levels.

Sotorasib tablets are administered PO QD with or without food for a treatment cycle of 21 days. Sotorasib tablets are formulated as PO, solid dosage form in a strength of 120 mg. The sotorasib dose used in the study is 960 mg per day.

The total study duration for an individual subject is approximately 6 years: 28-day screening, 6 to 12 months on treatment and up to approximately 46 cycles, SFU, and 5 years of LTFU from the last subject enrolled.

Concomitant administration of sotorasib with proton pump inhibitors (PPIs, such as omeprazole, pantoprazole, esomeprazole, lansoprazole, rabeprazole, and dexlansoprazole), or H2RA (such as famotidine, ranitidine, cimetidine, nizatidine, roxatidine, and lafutidine) is prohibited. If treatment with an acid-reducing agent cannot be avoided, sotorasib should be administered 4 hours before or 10 hours after a local antacid, such as sodium bicarbonate, calcium carbonate, aluminum hydroxide, and magnesium hydroxide.

The efficacy subgroup analysis will be performed for subjects with PD-L1 < 1%, STK11 co-mutation, and other co-occurring mutations of interest. For primary and final analyses, subgroups will be determined based on central data.

### Objectives and Endpoints

| **Objectives** | | **Endpoints** | |
|---|---|---|---|
| **Primary** | | | |
| | • To evaluate the tumor objective response rate (ORR) assessed by Response Evaluation Criteria in Solid Tumors (RECIST, Eisenhauer et al, 2009) 1.1 criteria in subjects who receive sotorasib at 960 mg daily (QD) whose tumors are programmed death-ligand 1 (PD-L1) Tumor Proportion Score (TPS) < 1% and/or harbor a serine/threonine kinase 11 (*STK11*) co-mutation, in a subgroup of subjects with PD-L1 < 1% and in a subgroup of subjects with *STK11* co-mutation. | | • Objective response (OR) (OR = complete response [CR] + partial response [PR]), measured by computed tomography (CT) or magnetic resonance imaging (MRI) and assessed per RECIST 1.1 per Blinded Independent Central Review (BICR) |

| **Key Secondary** | | | |
|---|---|---|---|
| | • To evaluate other measures of efficacy. | | • Disease control (CR + PR + stable disease [SD]) |
| | | | • Duration of response (DOR) |
| | | | • Time to response (TTR) |
| | | | • Progression-free survival (PFS) |
| | | | • Overall survival (OS) |
| | • To evaluate the safety and tolerability of sotorasib. | | • Treatment-emergent adverse events, treatment-related adverse events, and changes in vital signs, electrocardiogram [ECGs], and clinical laboratory tests. |
| | • Characterize the pharmacokinetics (PK) of sotorasib following administration as an oral tablet formulation | | • PK parameters of sotorasib including, but not limited to, maximum plasma concentration (Cₘₐₓ), time to achieve Cₘₐₓ (tₘₐₓ), and area under the plasma concentration-time curve (AUC) |

### Study Population

Subject inclusion criteria include the following:
- Untreated stage IV (per American Joint Committee on Cancer (AJCC) v8, see Amin et al., 2017) NSCLC.
   o Subjects who received adjuvant or neoadjuvant therapy are eligible if the adjuvant/neoadjuvant therapy was completed greater than 12 months prior to the development of metastatic disease.
- Pathologically documented, metastatic NSCLC with KRAS p.G12C mutation identified through molecular testing. KRAS p.G12C mutation must be performed in a Clinical Laboratory Improvement Amendments (CLIA) certified laboratory or equivalent.
- Age ≥ 18 years.
- PD-L1 TPS score < 1% as determined by pharm Dx DAKO 22C3 or Ventana SP263 IHC. If subjects do not have PD-L1 TPS score < 1%, STK11 loss of function mutation as determined by NGS must be present. Subjects with PD-L1 TPS score < 1% may also have presence of STK11 mutation.
- Subjects must be willing to provide archived tumor tissue samples (formalin-fixed paraffin-embedded [FFPE] sample collected within 5 years) or willing to undergo pretreatment tumor biopsy.
- Measurable disease per investigator interpretation using RECIST 1.1 criteria (Eisenhauer et al, 2009). Lesions previously radiated are not considered measurable unless they have progressed after radiation.
- Eastern Cooperative Oncology Group (ECOG, Oken et al., 1982) Performance Status of ≤ 1.
- Life expectancy of > 3 months, in the opinion of the investigator.
- Ability to take oral medications and willing to record daily adherence to investigational product.
- Adequate hematological laboratory assessments:
   ∘ Absolute neutrophil count (ANC) ≥ 1500 cells/µL
   ∘ Hemoglobin ≥ 9.0 g/dL
   ∘ Platelet count ≥ 75 000/µL
- Adequate renal laboratory assessments, defined as the following:
   ∘ Estimated glomerular filtration rate based on Modification of Diet in Renal Disease (MDRD) calculation ≥ 30 mL/min/1.73 m²
   ∘ Estimated glomerular filtration rate = 175 x serum creatinine^{-1.154} x age^{-0.203} x sex (0.742 if female) x race (1.210 if black)
- Adequate hepatic laboratory assessments, as follows:
   ∘ Aspartate aminotransferase (AST) ≤ 2.5 x upper limit of normal (ULN)
   ∘ Alanine aminotransferase (ALT) ≤ 2.5 x ULN
   ∘ Total bilirubin (TBL) ≤ 1.5 x ULN for subjects with documented Gilbert's syndrome or < 3.0 x ULN for subjects for whom the indirect bilirubin level suggests an extrahepatic source of elevation
- Adequate coagulation laboratory assessments, as follows:
   ∘ Prothrombin time (PT) or partial thromboplastin time (PTT) < 1.5 x ULN, OR
   ∘ International normalized ratio (INR) < 1.5 x ULN or within target range if on prophylactic anticoagulation therapy
- QTc ≤ 470 msec in females or ≤ 450 msec in males (based on average of screening triplicates)

Subject exclusion criteria include the following:
Disease Related
   - Mixed small-cell lung cancer and NSCLC histology.
   - Subject has received prior treatment for metastatic NSCLC. Subjects who receive adjuvant or neoadjuvant therapy are eligible if the adjuvant/neoadjuvant therapy was completed greater than 12 months prior to the development of metastatic disease.
Other Medical Conditions
   - History or presence of malignancy unless treated with curative intent and no evidence of disease ≥ 3 years with the following exceptions:
      ∘ Adequately treated non-melanoma skin cancer or lentigo maligna without evidence of disease
      ∘ Adequately treated cervical carcinoma in situ without evidence of disease
      ∘ Adequately treated breast ductal carcinoma in situ without evidence of disease
      ∘ Prostatic intraepithelial neoplasia without evidence of prostate cancer
      ∘ Adequately treated urothelial papillary noninvasive carcinoma or carcinoma in situ
   - Spinal cord compression, active brain metastases and/or carcinomatous meningitis. Subjects who have had brain metastases resected or have received whole brain radiation therapy ending at least 4 weeks (or stereotactic radiosurgery ending at least 2 weeks) prior to study day 1 are eligible if they meet all of the following criteria:
      ∘ No residual neurological symptoms
      ∘ No corticosteroid requirement to manage neurological symptoms
      ∘ Follow-up MRI performed within 30 days prior to enrollment shows no new lesions or enlarging lesion appearing
      ∘ No single lesion larger than 10 mm
   - Myocardial infarction within 6 months of study day 1, symptomatic congestive heart failure (New York Heart Association > Class II), unstable angina, or cardiac arrythmia requiring medication.
   - Gastrointestinal (GI) tract disease causing the inability to take oral medication, malabsorption syndrome, requirement for intravenous (IV) alimentation, uncontrolled inflammatory GI disease (e.g., Crohn's disease, ulcerative colitis).
   - Evidence of hepatitis infection based on the following results and/or criteria:
      ∘ Positive hepatitis B surface antigen (HepBsAg) (indicative of chronic hepatitis B or recent acute hepatitis B).
      ∘ Negative HepBsAg with a positive for hepatitis B core antibody (hepatitis B core antibody testing is not required for screening, however if this is done and is positive, then hepatitis B surface antibody [Anti-HBs] testing is necessary. Undetectable anti-HBs in this setting would suggest unclear and possible infection and requires exclusion).
      ∘ Positive hepatitis C virus antibody: Hepatitis C virus RNA by polymerase chain reaction (PCR) is necessary. Detectable Hepatitis C virus RNA renders the subject ineligible.
      ∘ Positive hepatitis B or C viral load: if above antibody/antigen testing is not able to be obtained, obtain hepatitis B or C viral load.
   - Uncontrolled pleural effusion, pericardial effusion, or ascites requiring recurrent drainage procedures at a frequency greater than monthly. Subjects with PleurX catheters in place may be considered for the study only with Medical Monitor approval.
   - Known positive test for human immunodeficiency virus (HIV).
   - Has a history of (non-infectious) pneumonitis that required steroids or has current pneumonitis.
   - Active infection within 2 weeks of study day 1 requiring therapeutic oral or IV antibiotics.
   - Major surgery within 28 days of study day 1.
   - Unresolved toxicities from prior anti-tumor therapy, defined as not having resolved to CTCAE version 5.0 grade 0 or 1, or to levels dictated in the eligibility criteria with the exception of alopecia (any grade allowed). Grade 2 or 3 toxicities from prior anti-tumor therapy that are considered irreversible (defined as having been present and stable for > 6 month), such as ifosfamide related proteinuria or neuropathy, may be allowed if they are not otherwise described in the exclusion criteria AND there is agreement to allow by both the investigator and sponsor.
Prior/Concomitant Therapy
   - Anti-tumor therapy (chemotherapy, antibody therapy, molecular targeted therapy, or investigational agent) within 12 months.
   - Therapeutic or palliative radiation therapy within 2 weeks of study day 1. Subjects must have recovered from all radiotherapy related toxicity to grade 1 or better.
   - Received radiation therapy to the lung that is > 30 Gy within 6 months of first dose of trial treatment.
   - Previous treatment with a covalent KRAS p.G12C inhibitor.
   - Use of known cytochrome P450 (CYP) 3A4 sensitive substrates or P-gp substrates, with a narrow therapeutic window, within 14 days or 5 half-lives of the drug or its major active metabolite, whichever is longer, prior to study day 1 that was not reviewed and approved by the principal investigator and the medical monitor.
   - Use of strong inducers of CYP3A4 (including herbal supplements such as St. John's wort) within 14 days or 5 half-lives, whichever is longer, prior to study day 1 that was not reviewed and approved by the principal investigator and the medical monitor.
   - Use of proton-pump inhibitors (PPIs) or histamine 2 (H2) receptor antagonists (H2RA) within 14 days or 5 half-lives of the drug or its major active metabolite, whichever is longer, prior to study day 1 that was not reviewed and approved by the principal investigator and the medical monitor.
Prior/Concurrent Clinical Study Experience
   - Currently receiving treatment in another investigational device or drug study, or less than 28 days since ending treatment on another investigational device or drug study(ies). Other investigational procedures while participating in this study are excluded.
Other Exclusions
   - Female subjects of childbearing potential unwilling to use protocol specified method of contraception (see Section 11.5) during treatment and for an additional 7 days after the last dose of sotorasib
   - Female subjects who are breastfeeding or who plan to breastfeed while on study through 7 days after the last dose of sotorasib.
   - Female subjects planning to become pregnant while on study through 7 days after the last dose of sotorasib.
   - Female subjects of childbearing potential with a positive pregnancy test assessed at Screening or day 1 by a highly sensitive urine or serum pregnancy test.
   - Male subjects with a female partner of childbearing potential who are unwilling to practice sexual abstinence (refrain from heterosexual intercourse) or use contraception during treatment and for an additional 7 days after the last dose of sotorasib.
   - Male subjects with a pregnant partner who are unwilling to practice abstinence or use a condom during treatment and for an additional 7 days after the last dose of sotorasib.
   - Male subjects unwilling to abstain from donating sperm during treatment and for an additional 7 days after the last dose of sotorasib.
   - Subject has known sensitivity to any of the products or components to be administered during dosing.
   - Subject likely to not be available to complete all protocol-required study visits or procedures, and/or to comply with all required study procedures (e.g., Clinical Outcome Assessments) to the best of the subject and investigator's knowledge.
   - Subject unable to receive both iodinated contrast for computed tomography (CT) scans and gadolinium contrast for MRI scans.
   - History or evidence of any other clinically significant disorder, condition or disease (with the exception of those outlined above) that, in the opinion of the investigator or physician, if consulted, would pose a risk to subject safety or interfere with the study evaluation, procedures or completion.

### REFERENCES

American Association for Cancer Research (AACR) Project GENIE Consortium. AACR Project GENIE: Powering Precision Medicine through an International Consortium. Cancer Discov. 2017;7(8):818-831.
Amin M. B., et al., AJCC Cancer Staging Manual, Eighth Edition. New York; Springer International Publishing; 2017.
An J, Yan M, Yu N, et al. Outcomes of patients with stage III non-small cell lung cancer (NSCLC) that harbor a STK11 mutation. J Clin Oncol. 2020;38(15 suppl):9033-9033.
Barbacid M. Ras genes. Annual Rev Biochem. 1987;56:779-827.
Arbour KC, Jordan E, Kim HR, et al. Effects of co-occurring genomic alterations on outcomes in patients with KRAS-mutant non-small cell lung cancer. Clin Cancer Res. 2018;24(2):334-340.
Atezolizumab Prescribing Information, revised April 2021 (available at https://www.gene.com/download/pdf/tecentriq_prescribing.pdf, accessed May 2021).
Biernacka A, Tsongalis PD, Peterson JD, et al. The potential utility of re-mining results of somatic mutation testing: KRAS status in lung adenocarcinoma. Cancer Genet. 2016;209(5):195-198.
Blumenthal, SW, Karuri SW, Zhan H, et al. Overall Response Rate, Progression-Free Survival, and Overall Survival With Targeted and Standard Therapies in Advanced Non-Small-Cell Lung Cancer: US Food and Drug Administration Trial-Level and Patient-Level Analyses. J Clin Oncol. 2015;Mar 20; 33(9): 1008-1014.
Borghaei H, Paz-Ares L, Horn L, et al. Nivolumab versus docetaxel in advanced nonsquamous non-small-cell lung cancer. N Engl J Med. 2015;373(17):1627-1639.
Canon, J, Rex, K, Saiki, AY, et al. The clinical KRAS(G12C) inhibitor AMG 510 drives antitumour immunity. Nature. 2019;575;217-223.
Clarke J, Wang X, and Ready N. Surrogate clinical endpoints to predict overall survival in non-small cell lung cancer trials-are we in a new era? Transl Lung Cancer Res. 2015;Dec; 4(6): 804-808.
Cully M, Downward J. SnapShot: Ras Signaling. Cell. 2008;133:1292.
Davis AA, Patel VG. The role of PD-L1 expression as a predictive biomarker: an analysis of all US Food and Drug Administration (FDA) approvals of immune checkpoint inhibitors. J Immunother Cancer. 2019;7(1):278.
Eisenhauer EA, Therasse P, Bogaerts J, et al. New response evaluation criteria in solid tumours: revised RECIST guideline (version 1.1). Eur J Cancer. 2009; 45: 228-247.
Ettinger DS, Wood DE, Aggarwal C, et al. NCCN Guidelines Insights: Non-small Cell Lung Cancer, Version 1.2020. J Natl Compr Canc Netw. 2019;17(12):1464-1472.
Fernández-Medarde A, Santos E. Ras in cancer and developmental diseases. Genes Cancer. 2011;2(3):344-358.
Fusco MJ, West H, Walko CM. Tumor Mutation Burden and Cancer Treatment. JAMA Oncol. 2021;7(2):316.
Gainor JF, Varghese AM, Ou SH, et al. ALK rearrangements are mutually exclusive with mutations in EGFR or KRAS: an analysis of 1,683 patients with non-small cell lung cancer. Clin Cancer Res. 2013;19(15):4273-4281.
GLOBOCAN 2018 - Lung. Available at http://gco.iarc.fr/today/home. Accessed August 2019.
Goeman F, De Nicola F, Scalera S, et al. Mutations in the KEAP1-NFE2L2 pathway define a molecular subset of rapidly progressing lung adenocarcinoma. J Thorac Oncol. 2019;14(11):1924-1934.
Gridelli C, Catalono PJ, Meropol NJ, et al. Safety and efficacy of bevacizumab plus standard-of-care treatment beyond disease progression in patients with advanced non-small cell lung cancer. The AvaALL randomized clinical trial. JAMA Onc. 2018;4(12):e183486.
Herbst RS, Baas P, Kin DW, et al. Pembrolizumab versus docetaxel for previously treated, PD-L1-positive, advanced non-small-cell lung cancer (KEYNOTE-010): a randomised controlled trial. Lancet. 2016;387:1540-1550.
Herbst RS, O'Neill, Fehrenbacher L, et al. Phase II study of efficacy and safety of bevacizumab in combination with chemotherapy or erlotinib compared with chemotherapy alone for treatment of recurrent or refractory non-small-cell lung cancer. J Clin Oncol. 2007;25(30):4743-4750.
Hong DS, Fakih MG, Strickler JH, et al. KRASG12C Inhibition with Sotorasib in Advanced Solid Tumors. N Engl J Med. 2020;383:1207-1217.
Jeong Y, Hellyer JA, Stehr H, et al. Role of KEAP1/NFE2L2 mutations in the chemotherapeutic response of patients with non-small cell lung cancer. Clin Cancer Res. 2020;26(1):274-281.
Jones RP, Sutton PA, Evans JP, et al. Specific mutations in KRAS codon 12 are associated with worse overall survival in patients with advanced and recurrent colorectal cancer. Br J Cancer. 2017;116(7):923-929.
Martorell MP, Huerta M, Compañ Quilis A, et al. Coexistence of EGFR, KRAS, BRAF, and PIK3CA Mutations and ALK Rearrangement in a Comprehensive Cohort of 326 Consecutive Spanish Nonsquamous NSCLC Patients. Clin Lung Cancer. 2017;Nov;18(6):e395-e402.
McCormick F. K-Ras protein as a drug target. J Mol Med (Berl). 2016;94(3):253-258.
McCormick F. Progress in targeting RAS with small molecule drugs. Biochem J. 2019;476 365-374.
Oken M, Creech R, Tormey D, et al. Toxicity and response criteria of the Eastern Cooperative Oncology Group. Am J Clin Oncol. 1982;5:649-655.
Ostrem JM, Peters U, Sos ML, Wells JA, Shokat KM. K-Ras (G12C) inhibitors allosterically control GTP affinity and effector interactions. Nature. 2013;503:548-551*.*
Pavan A, Zulato E, Calvetti L, et al. Plasma next-generation sequencing (NGS) in advanced non-small cell lung cancer (aNSCLC) patients (pts) treated with immune checkpoint inhibitors (ICIs): impact of STK11 and TP53 mutations on outcome. J Clin Oncol. 2020;38(15 suppl):3046-3046.
Prembrolizumab Prescribing Information, revised November 2020, https://www.merck.com/product/usa/pi_circulars/k/keytruda/keytruda_pi.pdf; accessed January 2020.
Planchard S, Popat K, Kerr S, et al. Metastatic non-small cell lung cancer: ESMO Clinical Practice Guidelines for diagnosis, treatment and follow-up. Ann Oncol. 2018;29 (Supplement 4):iv192-iv237.
Prior IA, Lewis PD, Mattos C. A comprehensive survey of Ras mutations in cancer. Cancer Res. 2012;72 (10):2457-2467.
Ricciuti B, Arbour KC, Lin JJ, et al. Effect of STK11 mutations on efficacy of PD-1 inhibition in non-small cell lung cancer (NSCLC) and dependence on KRAS mutation status. J Clin Oncol. 2020;38(15 suppl):e15113-e15113.
Rittmeyer A, Barlesi F, Waterkamp D, et al. Atezolizumab versus docetaxel in patients with previously treated non-small-cell lung cancer (OAK): a phase 3, open-label, multicentre randomised controlled trial. Lancet. 2017;380:255-265.
Román M, Baraibar I, López I, et al. KRAS oncogene in non-small cell lung cancer: clinical perspectives on the treatment of an old target. Mol Cancer. 2018;17(1):33.
Scheffler M, Ihle MA, Hein R, et al. K-ras mutation subtypes in NSCLC and associated co-occuring mutations in other oncogenic pathways. J Thorac Oncol. 2019;14(4):606-616.
Simanshu DK, Nissley DV, McCormick F. RAS proteins and their regulators in human disease. Cell. 2017;170:17-33.
Solis LM, Behrens C, Dong W, et al. Nrf2 and KEAP1 abnormalities in non-small cell lung carcinoma and association with clinicopathologic features. Clin Cancer Res. 2010;16(14):3743-3753.
Surveillance, Epidemiology, and End Results (SEER) Program (www.seer.cancer.gov) SEER*Stat Database: Incidence - SEER 21 Regs Limited-Field Research Data + Hurricane Katrina Impacted Louisiana Cases, Nov 2018 Sub (2000-2016) <Katrina/Rita Population Adjustment> - Linked To County Attributes - Total U.S., 1969-2017 Counties, National Cancer Institute, DCCPS, Surveillance Research Program, released April 2019, based on the November 2018 submission. Accessed 10 February 2020.
Tamiya Y, Zenke Y, Matsumoto S, et al. Therapeutic impact of mutation subtypes and concomitant STK11 mutations in KRAS-mutated non-small cell lung cancer (NSCLC): A result of nationwide genomic screening project (LC-SCRUM-Japan). J Clin Oncol. 2020;38(15_suppl):9589-9589.
Tian Y, Wu K, Liu Q, et al. Modification of platinum sensitivity by KEAP1/NRF2 signals in non-small cell lung cancer. J Hematol Oncol. 2016;9(1):83.
Uba R, Raez LE, Dumais K, et al. Serine/threonine kinase 11 (STK11) mutations and immunotherapy resistance in patients with non-small cell lung cancer. J Clin Oncol. 2020;38(15 suppl):e15055-e15055.
Van Cutsem E, Cervantes A, B. Nordlinger B, Arnold D. ESMO Guidelines Working Group. Metastatic colorectal cancer: ESMO Clinical Practice Guidelines for diagnosis, treatment and follow-up. Ann Oncol. 2014:25 (Supplement 3):iii1-iii9.
World Health Organization. 2018 Statistics. Available at https://www.who.int/en/news-room/fact-sheets/detail/cancer. Accessed October 2020.

All references, for example, a scientific publication or patent application publication, cited herein are incorporated herein by reference in their entirety and for all purposes to the same extent as if each reference was specifically and individually indicated to be incorporated by reference in its entirety for all purposes.

### Clauses

1. A method of treating *KRAS G12C* mutated non-small cell lung cancer in a subject in need thereof, comprising administering to the subject a total daily dose of 960 mg of a compound of Formula I 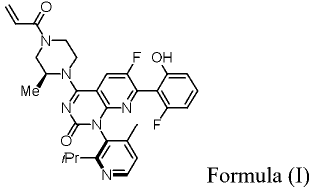 or a pharmaceutically acceptable salt thereof, wherein the subject has a PD-L1 tumor proportion score of less than 50%.
2. The method according to clause1, wherein the PD-L1 tumor proportion score is equal or more than 1% and less than 50%.
3. The method according to clause1, wherein the PD-L1 tumor proportion score is less than 1%.
4. A method of treating *KRAS G12C* mutated non-small cell lung cancer in a subject in need thereof, comprising administering to the subject a total daily dose of 960 mg of a compound of Formula I 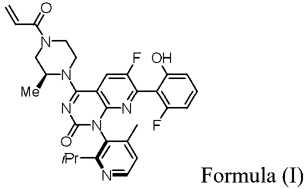 or a pharmaceutically acceptable salt thereof, wherein the subject is positive for a loss-of-function mutation of *STK11.*
5. The method of clause 4, wherein the subject is positive for a loss-of-function mutation of *KEAP1.*
6. The method of clause 4, wherein the subject is positive for a wild-type of *KEAP1.*
7. A method of treating *KRAS G12C* mutated non-small cell lung cancer in a subject in need thereof, comprising administering to the subject a total daily dose of 960 mg of a compound of Formula I 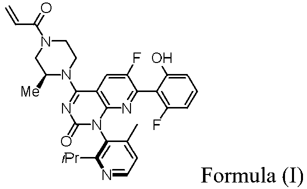 or a pharmaceutically acceptable salt thereof, wherein (i) the subject has a PD-L1 tumor proportion score of less than 50% or (ii) the subject is positive for a loss-of-function mutation of *STK11.*
8. A method of treating *KRAS G12C* mutated non-small cell lung cancer in a subject in need thereof, comprising administering to the subject a total daily dose of 960 mg of a compound of Formula I 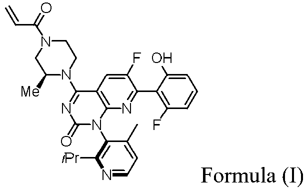 or a pharmaceutically acceptable salt thereof, wherein (i) the subject has a PD-L1 tumor proportion score of less than 50% and (ii) the subject is positive for a loss-of-function mutation of *STK11.*
9. A method of treating *KRAS G12C* mutated non-small cell lung cancer in a subject in need thereof, comprising administering to the subject a total daily dose of 960 mg of a compound of Formula I 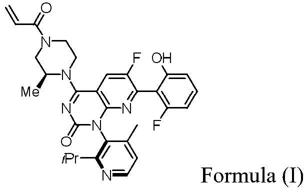 or a pharmaceutically acceptable salt thereof, wherein (i) the subject has a PD-L1 tumor proportion score of less than 50%, (ii) the subject is positive for a loss-of-function mutation of *STK11,* or (iii) the subject has a PD-L1 tumor proportion score of less than 50% and the subject is positive for a loss-of-function mutation of *STK11.*
10. The method according to any one of clauses 7-9, wherein the PD-L1 tumor proportion score is equal or more than 1% and less than 50%.
11. The method according to any one of clauses 7-9, wherein the PD-L1 tumor proportion score is less than 1%.
12. The method of any one of clauses 7-11, wherein the subject is positive for a loss-of-function mutation of *KEAP1.*
13. The method of any one of clauses 7-11, wherein the subject is positive for a wild-type of *KEAP1.*
14. The method according to any one of clauses 1-13, wherein the subject has received no prior anticancer systemic therapy.
15. The method according to any one of clauses 1-13, wherein the subject has received at least one prior anticancer systemic therapy.
16. The method according to any one of clauses 1-13, wherein the subject has received one prior anticancer systemic therapy.
17. The method according to any one of clauses 1-13, wherein the subject has received two prior anticancer systemic therapies.
18. The method according to clauses 1-13, wherein the subject as received three prior anticancer systemic therapies.
19. The method according to clause 15 or clause 16, wherein the one prior anticancer systemic therapy is an anti-PD1 or anti-PD-L1 immunotherapy.
20. The method according to clause 15 or clause 16, wherein one prior anticancer systemic therapy is a platinum-based chemotherapy.
21. The method according to any one of clauses 1-20, wherein the non-small cell lung cancer is stage IV non-small cell lung cancer.
22. The method according to any one of clauses 1-20, wherein the non-small cell lung cancer is locally advanced or metastatic non-small cell lung cancer.
23. The method according to any one of clauses 1-22, wherein the subject has an Eastern Cooperative Oncology Group (ECOG) Performance Status of 1 or 0.
24. The method according to any one of clauses 1-23, wherein the subject is 18 years or older.
25. The method according to any one of clauses 1-24, wherein the compound is administered as a free base.
26. The method according to any one of clauses 1-25, wherein the compound is administered as a pharmaceutical composition comprising the compound and a pharmaceutically acceptable excipient.
27. The method according to clause 26, wherein the pharmaceutical composition is a solid dosage form.
28. The method according to clause 26 or clause 27, wherein the pharmaceutical composition is for oral administration.
29. The method according to any one of clauses 26-28, wherein the pharmaceutical composition is a tablet.
30. The method according to any one of clauses 1-29, wherein total daily dose of the compound is administered once daily.

## Claims

1. A compound of Formula I 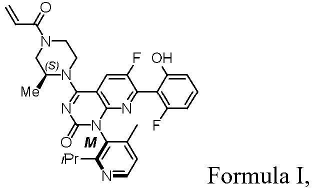 or a pharmaceutically acceptable salt thereof, for use in treating KRAS *G12C* mutated non-small cell lung cancer in a subject in need thereof, wherein the use comprises administering to the subject a total daily dose of 960 mg of the compound of Formula I and wherein the subject has not received a prior systemic platinum-based chemotherapy.

2. A method of identifying a subject having non-small cell lung cancer as sensitive to treatment with a compound of Formula I 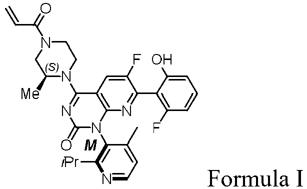 or a pharmaceutically acceptable salt thereof, comprising (i) assaying a sample obtained from the subject for a *KRAS G12C* mutation and (ii) determining whether the subject has received a prior systemic platinum-based chemotherapy, wherein the subject is identified as sensitive to treatment with a compound of Formula I or a pharmaceutically acceptable salt thereof, when the sample is positive for a *KRAS G12C* mutation and the subject has not received a prior systemic platinum-based chemotherapy.

3. A compound of Formula I 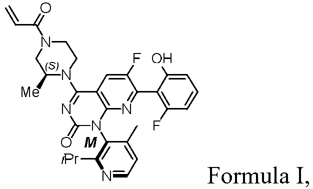 or a pharmaceutically acceptable salt thereof, for use in a method of treatment as determined for a subject having non-small cell lung cancer, comprising (i) assaying a sample obtained from the subject for a *KRAS G12C* mutation and (ii) determining whether the subject has received a prior systemic platinum-based chemotherapy, when the sample is positive for a *KRAS G12C* mutation and the subject has not received a prior systemic platinum-based chemotherapy.

4. The method according to claim 2, wherein the treatment comprises administering a total daily dose of 960 mg of the compound of Formula I or a pharmaceutically acceptable salt thereof to the subject.

5. The method according to claim 3, wherein the treatment comprises administering a total daily dose of 960 mg of the compound of Formula I or a pharmaceutically acceptable salt thereof to the subject.

6. The compound for use or the method according to any one of claim 1 to 5, wherein the prior platinum-based chemotherapy comprises administration of a compound comprising platinum.

7. The compound for use or the method according to claim 6, wherein the compound comprising platinum is cisplatin or carboplatin.

8. The compound for use or the method according to any one of claims 1 to 7, wherein the subject is 18 years or older.

9. The compound for use or the method according to any one of claims 1 to 8, wherein the compound of Formula I is administered as a free base.

10. The compound for use or the method according to any one of claims 1 to 9, wherein the compound of Formula I is administered as a pharmaceutical composition comprising the compound and a pharmaceutically acceptable excipient.

11. The compound for use or the method according to claim 10, wherein the pharmaceutical composition is a solid dosage form.

12. The compound for use or the method according to claim 11, wherein the pharmaceutical composition is for oral administration.

13. The compound for use or the method according to any one of claims 10 to 12, wherein the pharmaceutical composition is a tablet.

14. The compound for use or the method according to claim 13, wherein the tablet comprises a fraction of the total daily dose of the compound of Formula I.

15. The compound for use or the method according to any one of claims 1 and 4 to 14, wherein the total daily dose of the compound is administered once daily.
